(19) Europäisches Patentamt
European Patent Office
Office européen des brevets

(11) Veröffentlichungsnummer: **0 117 448**
**B1**

(12) **EUROPÄISCHE PATENTSCHRIFT**

(45) Veröffentlichungstag der Patentschrift: **03.09.86**

(21) Anmeldenummer: **84100935.0**

(22) Anmeldetag: **31.01.84**

(51) Int. Cl.⁴: **C 07 C 99/00**, C 07 C 101/00, C 07 C 143/68, C 07 C 149/243, C 07 C 149/43

(54) **Verfahren zur Herstellung von N-alkylierten Aminosäuren und deren Estern.**

(30) Priorität: **02.02.83 DE 3303344**

(43) Veröffentlichungstag der Anmeldung: **05.09.84 Patentblatt 84/36**

(45) Bekanntmachung des Hinweises auf die Patenterteilung: **03.09.86 Patentblatt 86/36**

(84) Benannte Vertragsstaaten: **AT BE CH DE FR GB IT LI LU NL SE**

(56) Entgegenhaltungen:
**DE-B-1 275 052**
**FR-A-1 586 146**
**GB-A-1 572 185**

(73) Patentinhaber: **HOECHST AKTIENGESELLSCHAFT, Postfach 80 03 20, D-6230 Frankfurt am Main 80 (DE)**

(72) Erfinder: **Urbach, Hansjörg, Dr., Le Lavandoustrasse 41, D-6242 Kronberg/Taunus (DE)**
Erfinder: **Henning, Rainer, Dr., Rotenhofstrasse 31, D-6234 Hattersheim am Main (DE)**

EP 0 117 448 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Verfahren zur Herstellung von N-alkylierten Aminosäuren und deren Estern

Die Erfindung betrifft ein Verfahren zur Herstellung von Verbindungen der Formel I

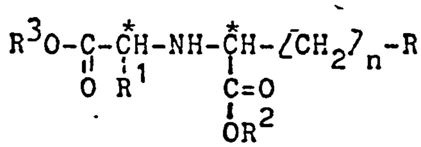

(I)

in welcher

n = 1 oder 2 ist,

R Wasserstoff, Carboxy, Carbamoyl,

einen gegebenenfalls substituierten aliphatischen Rest mit 2-8 C-Atomen,

einen gegebenenfalls substituierten cycloaliphatischen Rest mit 3 -9 C-Atomen, einen gegebenenfalls substituierten aromatischen Rest mit 6-12 C-Atomen, einen Rest $OR^4$ oder $SR^4$, worin

$R^4$ für einen gegebenenfalls substituierten aliphatischen Rest mit 1-4 C-Atomen, für einen gegebenenfalls substituierten aromatischen Rest mit 6-12 C-Atomen oder einen gegebenenfalls substituierten heteroarcmatischen Rest mit 5-10 Ringatomen steht, oder

einen gegebenenfalls substituierten heteroaromatischen Rest mit 5-10 Ringatomen,

$R^1$ Wasserstoff,

einen gegebenenfalls substituierten aliphatischen Rest mit 1-6 C-Atomen,

einen gegebenenfalls substituierten cycloaliphatischen Rest mit 3-9 C-Atomen,

einen gegebenenfalls substituierten cycloaliphatischaliphatischen Rest mit 4-13 C-Atomen, einen gegebenenfalls substituierten aromatische Rest mit 6-12 C-Atomen,

einen gegebenenfalls substituierten araliphatis hen Rest mit 7-16 C-Atomen,

einen gegebenenfalls substituierten heteroaromatischen Rest mit 5-10 Ringatomen oder

die Seitenkette einer gegebenenfalls geschützten natürlich vorkommenden α-Aminosäure bedeuten,

$R^2$ und $R^3$ gleich oder verschieden sind und Wasserstoff,

einen gegebenenfalls substituierten aliphatischen Rest mit 1-6 C-Atomen,

einen gegebenenfalls substituierten cycloaliphatischen Rest mit 3-9 C-Atomen,

einen gegebenenfalls substituierten cycloaliphatischaliphatischen Rest mit 4-12 C-Atomen,

einen gegebenenfalls substituierten aromatischen Rest mit 6-12 C-Atomen oder

einen gegebenenfalls substituierten araliphatischen Rest mit 7-16 C-Atomen

bedeuten,

das dadurch gekennzeichnet ist, daß man Verbindungen der Formeln II bzw. III

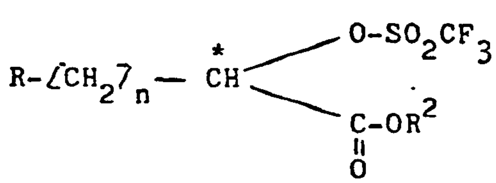

(II)

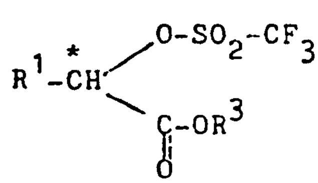

(III)

in welchen n, R, $R^1$, $R^2$ und $R^3$ die oben genannten Bedeutungen haben, mit Verbindungen der Formeln IV bzw.V

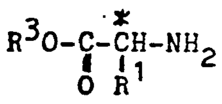

(IV)

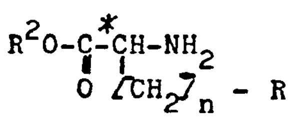

(V)

in denen R, $R^1$, $R^2$, $R^3$ und n die oben genannten Bedeutungen haben, umsetzt, gegebenenfalls Estergruppen hydrolytisch oder hydrogenolytisch abspaltet oder gegebenenfalls freie Carboxygruppen in an sich bekannter Weise verestert.

Aus der Literatur (z.B. aus der US-PS 4 350 704 und den EP-A 49 605 und 46 953) sind Verfahren zur Herstellung von Verbindungen der Formel I durch Umsetzung von -Halogencarbonsäureestern bzw. der entsprechenden Tosyloxy- oder Mesyloxy-Verbindungen mit Aminoestern bekannt. Die Umsetzung erfordert eine erhöhte Reaktionstemperatur. Die Ausbeuten sind auf Grund der drastischen Reaktionsbedingungen, die zu Nebenreaktionen führen, gering. Oft ist eine Katalyse mit Silberionen, wie z.B. bei der Umsetzung der α-Halogen-Verbindungen, notwendig, die die Ausbeute verbessern kann, jedoch kostenintensiver ist. Desweilen erhält man racemische Produkte, wenn optisch aktive α-Halogencarbonsäureester eingesetzt werden.

In einem weiteren Verfahren, das u.a. Gegenstand der deutschen Patentanmeldung P 32 26 768.1 ist, werden α-Ketoester mit Aminosäureestern umgesetzt und die entstehenden Iminoverbindungen mit verschiedenen Reduktionsmitteln reduziert. Bei der Verwendung von Natriumcyanborhydrid ist die Aufarbeitung durch das Entstehen von Blausäure aufwendig. Auch beim Einsatz optisch reiner Aminosäureester werden nach diesem Verfahren nur Diastereomerengemische erhalten, die gegebenenfalls auf aufwendige Weise aufgetrennt werden.

Das erfindungsgemäße Verfahren besitzt die angeführten Nachteile nicht.

Eine bevorzugte Ausführungsform ist dadurch gekennzeichnet, daß Verbindungen der Formel I hergestellt werden, in der n = 1 oder 2 ist

R Wasserstoff,
Alkyl mit 2-8 C-Atomen,
Alkenyl mit 2-6 C-Atomen,
Cycloalkyl mit 3-9 C-Atomen,
Aryl mit 6-12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Acyl-amino, vorzugsweise ($C_1$-$C_4$)-Alkanoylamino, Methylendioxy, Carboxy, Cyano und/oder Sulfamoyl mono-, dioder trisubstituiert sein kann,
Alkoxy mit 1-4 C-Atomen,
Aryloxy mit 6-12 C-Atomen,
das wie oben bei Aryl beschrieben substituiert sein kann,
mono bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)-alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_1$-$C_4$)-Alkoxycarbonyl)
($C_1$-$C_{12}$)-Aryloxy-($C_1$-$C_4$)-alkyl,

das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,
$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6-12 C-Atomen, das wie oben bei R beschrieben substituiert sein kann,
($C_6$-$C_{12}$)-Aryl-($C_1$-$C_4$)-alkyl oder ($C_7$-$C_{13}$)-Aroyl-($C_1$ oder $C_2$)alkyl
die beide wie das vorstehende Aryl substituiert sein können
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann
oder
die Seitenkette einer gegebenenfalls geschützten natürlich vorkommenden α-Aminosäure $R^1$-CH($NH_2$)-COOH bedeuten,
$R^2$ und $R^3$ gleich oder verschieden sind und Wasserstoff,
Alkyl mit 1-6 C-Atomen,
Alkenyl mit 2-6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl
Aryl mit 6-12 C-Atomen,
($C_6$-$C_{12}$) Aryl-($C_1$-$C_4$)-alkyl,
($C_3$-$C_9$) Cycloalkyl oder
($C_3$-$C_9$)-Cycloalkyl-($C_1$-$C_4$)-alkyl
bedeuten.

Besonders bevorzugt ist eine Ausführungsform, die dadurch gekennzeichnet ist, daß Verbindungen der Formel I hergestellt werden, in der
n = 1 oder 2 ist,
R ($C_2$ bis $C_6$)-Alkyl, ($C_2$ bis $C_6$)-Alkenyl, ($C_3$ bis $C_9$)-Cycloalkyl,Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$ bis $C_{12}$)-Aryl-($C_1$-$C_4$)-alkoxycarbonylamino-($C_1$-$C_4$)-alkyl,($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino und/oder Methylendioxy mono-, di- oder trisubstituiert sein kann, oder 3-Indolyl, insbesondere Ethyl, Cyclohexyl, tert. Butoxycarbonylamino-($C_1$-$C_4$)-alkyl, Benzoyloxycarbonyl-amino-($C_1$-$C_4$)-alkyl oder Phenyl, das durch Phenyl, ($C_1$ bis $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkyl-amino, Nitro und/oder Methylendioxy mono- oder disubstituiert oder im Falle von Methoxy, trisubstituiert sein kann, bedeutet,
$R^1$ Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_3$ bis $C_9$)-Cycloalkyl, ($C_5$ bis $C_9$)-Cyclo-alkenyl, ($C_3$ bis $C_7$)-Cycloalkyl-($C_1$ bis $C_4$)-alkyl, ($C_6$ bis $C_{12}$)-Aryl oder teilhydriertes Aryl, das jeweils durch ($C_1$ bis $C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl die beide wie vorstchend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$ bis $C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeuten und
$R^2$ und $R^3$ gleiche oder verschiedene Reste Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl, insbesondere aber Wasserstoff, ($C_1$ bis $C_4$)-Alkyl oder Benzyl bedeuten. Unter Aryl ist hier wie im folgenden vorzugsweise gegebenenfalls substituiertes Phenyl, Biphenylyl oder Naphthyl zu verstehen. Entsprechendes gilt für von Aryl abgeleitete Reste wie Aryloxy, Arylthio. Unter Aroyl wird insbesondere Benzoyl verstanden. Aliphatische Reste können geradkettig oder verzweigt sein.

Unter einem mono- bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b] thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl

verstanden. Diese Reste können auch teilweise oder vollständig hydriert sein.

Natürlich vorkommende α-Aminosäuren sind z.B. in Houben-Weyl, Methoden der Organischen Chemie, Bd. XV/1 und XV/2 beschrieben.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp, His oder Hyp, sind als Schutzgruppen die in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder ($C_1$ - $C_6$)-Alkanoyl bevorzugt. Als O-Schutzgruppen für Tyrosin kommen bevorzugt ($C_1$-$C_6$)-Alkyl, insbesondere Methyl oder Ethyl in Frage.

Nach dem erfindungsgemäßen Verfahren können, je nachdem, welche chiralen Ausgangsverbindungen verwendet wurden, Verbindungen der Formel I erhalten werden, in denen das bei dieser $S_N2$-Reaktion entstandene Chiralitätszentrum in der S- oder R-Konfiguration oder racemisch vorliegt.

Die beim erfindungsgemäßen Verfahren stattfindende Reaktion verläuft stereochemisch eindeutig. Diese Tatsache wird auch durch die Untersuchungen des stereochemischen Verlaufs der Reaktion von α -Trifluormethansulfonyloxy-carbonsäureestern mit optisch aktiven Aminen bestätigt (Effenberger et al., Angew. Chem. 95 (1983) 50).

Das nachstehende Schema erläutert den stereochemischen Verlauf der Reaktion beim erfindungsgemäßen Verfahren:

Ausgangsverbindungen

| Ausgangsverbindungen | | a b<br>$R^3O_2C$-CH-NH-CH-/$CH_2$/$_\eta$-R<br>(I) $R^1$ $CO^2$ $R^2$ |
|---|---|---|
| (R)-IV + (R)-II | → | ($R_a$, $S_b$)-I |
| (R)-IV + (S)-II | → | ($R_a$, $R_b$)-I |
| (S)-IV + (R)-II | → | ($S_a$, $S_b$)-I |
| (S)-IV + (S)-II | → | ($S_a$, $R_b$)-I |
| (R)-III + (R)-V | → | ($S_a$, $R_b$)-I |
| (R)-III + (S)-V | → | ($S_a$, $S_b$)-I |
| (S)-III + (R)-V | → | ($R_a$, $R_b$)-I |
| (S)-III + (S)-V | → | ($R_a$, $S_b$)-I |

Besonders vorteilhaft können die folgenden Verbindungen nach dem erfindungsgemäßen Verfahren erhalten werden. N-(1-S-Carbethoxy-3-phenylpropyl)L-S-alaninbenzylester

N-(1-R-Carbethoxy-3-phenylpropyl)-S-alaninbenzylester
N-(1-S-Carbethoxy-3-phenylpropyl)-R-alaninbenzylester
N-(1-R-Carbethoxy-3-phenylpropyl)-R-alaninbenzylester
N-(1-R,S-Carbethoxy-3-phenylpropyl)-S-alaninbenzylester
N-(1-R,S-Carbethoxy-3-phenylpropyl)-R-alaninbenzylester
N-(1-S-Carbethoxy-3-phenylpropyl)-R,S-alaninbenzylester
N-(1-R-Carbethoxy-3-phenylpropyl)-R,S-alaninbenzylester
N-(1-R,S-Carbethoxy-3-phenylpropyl)-R,S-alaninbenzylester
$N_\alpha$ -(1-S-Carbethoxy-3-phenylpropyl)-$N_\varepsilon$ -benzyloxycarbonyl-S-lysin-tert.-butylester
$N_\alpha$ -(1-S-Carbethoxy-3-phenylpropyl)-$N_\varepsilon$ -tert.-butoxycarbonyl-S-lysinbenzylester
N-(1-S-Carbethoxy-3-phenylpropyl)-O-ethyl-S-tyrosinbenzylester
N-(1-S-Carbethoxy-3-phenylpropyl)-O-methyl-S-tyrosinbenzylester
N-(1-S-Carbethoxy-3-phenylpropyl)-S-tyrosinbenzylester
N-(1-S-Carbethoxy-3-[4-fluorphenyl]-propyl)-S-alaninbenzylester
N-(1-S-Carbethoxy-3-[4-methoxyphenyl]-propyl)-S-alaninbenzylester
N-(1-S-Carbethoxy-3-[4-chlorphenyl]-propyl)-S-alaninbenzylester
N-(1-S-Carbethoxy-3-[2-methylphenyl]-propyl)-S-alaninbenzylester
N-(1-S-Carbethoxy-3-[3,4-dimethoxyphenyl]-propyl)-S-alaninbenzylester
N-(1-S-Carbethoxy-butyl)-S-alaninbenzylester
N-(1-S-Carbethoxy-3-[cyclohexyl]-propyl)-S-alaninbenzylester

N-(1-S-Carbethoxy-3-[4-phenylphenyl]-propyl)-S-alaninbenzylester
N-(1-S-Carbethoxy-3-[4-fluorphenyl]-propyl)-O-methyl-S-tyrosinbenzylester
N-(1-S-Carbethoxy)-[4-fluorphenyl]-propyl)-O-ethyl-S-tyrosinbenzylester
N-(1-S-Carbethoxy-3-[4-methoxyphenyl]-propyl)-O-ethyl-S-tyrosinbenzylester
N-(1-S-Carbethoxy-3-[4-chlorphenyl] -propyl)-O-ethyl-Styrosinbenzylester
N-(1-S-Carbethoxy-3-[2-methylphenyl]-propyl)-O-ethyl-S-tyrosinbenzylester
N-(1-S-Carbethoxy-3-[3,4-dimethoxyphenyl]-propyl)-O-ethyl-S-tyrosinbenzylester
N-(1-S-Carbethoxy-butyl)-O-ethyl-S-tyrosinbenzylester
N-(1-S-Carbethoxy-3- cyclohexyl -propyl)-O-ethyl-S-tyrosinbenzylester
N-(1-S-Carbethoxy-3-[4-methoxyphenyl]-propyl)-O-methyl-S-tyrosinbenzylester
N-(1-S-Carbethoxy-3-[4-chlorphenyl]-propyl)-O-methyl-S-tyrosinbenzylester
N-(1-S-Carbethoxy-3-[2-methylphenyl]-propyl)-O-methyl-S-tyrosinbenzylester
N-(1-S-Carbethoxy-3-[3,4-dimethoxyphenyl]-propyl)-O-methyl-S-tyrosinbenzylester
N-(1-S-Carbethoxy-butyl)-O-methyl-S-tyrosinbenzylester
N-(1-S-Carbethoxy-3-[cyclohexyl]-propyl)-O-methyl-S-tyrosin-benzylester
$N_\alpha$ -(1-S-Carbethoxy-3-[4-fluorphenyl]-propyl)-$N_\varepsilon$ -benzyloxycarbonyl-S-lysin-tert.-butylester
$N_\alpha$ -(1-S-Carbethoxy-3-[4-methoxyphenyl]-propyl)-$N_\varepsilon$ -benzyloxycarbonyl-S-lysin-tert.-butylester
$N_\alpha$ -(1-S-Carbethoxy-3-[4-chlorphenyl]-propyl)-$N_\varepsilon$ -benzyloxycarbonyl-S-lysin-tert.-butylester
$N_\alpha$ -(1-S-Carbethoxy-3-[2-methylphenyl]-propyl)-$N_\varepsilon$ -benzyloxycarbonyl-S-lysin-tert.-butylester
$N_\alpha$ -(1-S-Carbethoxy-3-/3,4-dimethoxy-phenyl]-propyl
$N_\alpha$ -benzyloxycarbonyl-S-lysin-tert.-butylester
$N_\alpha$ -(1-S-Carbobenzyloxy-ethyl)-$N_\varepsilon$ -tert.-butoxycarbonyl- S-lysinethylester
$N_\alpha$-(1-S-Carbo-tert.butoxy-ethyl)-$N_\varepsilon$-benzyloxycarbonyl-S-lysinethylester
$N_\alpha$ -(1-S-Carbethoxy-butyl)-$N_\varepsilon$ -benzyloxycarbonyl-S-lysintert.butylester
$N_\alpha$ -(1-S-Carbethoxy-3-[cyclohexyl]-propyl)-$N_\varepsilon$ -benzyloxycarbonyl-S-lysin-tert.butylester
$N_\alpha$-(1-S-Carbo tert.butoxy-2-[4-ethoxyphenyl]-ethyl)-$N_\varepsilon$-benzyl-oxycarbonyl-S-lysin-ethylester
$N_\alpha$-(1-S-Carbotert.butoxy-2-[4-methoxyphenyl]-ethyl)-$N_\varepsilon$ -benzyloxycarbonyl-S-lysin-ethylester

Die Erfindung betrifft auch Verbindungen der Formeln II und III, in welchen
n = 2 ist,
R ($C_1$ bis $C_6$)-Alkyl, ($C_2$ bis $C_6$)-Alkenyl, ($C_3$ bis $C_9$)-Cycloalkyl, Amino($C_1$-$C_4$)-alkyl, ($C_2$-$C_5$)-Acylamino-($C_1$-$C_4$)ethyl, ($C_7$-$C_{19}$)-Aroylamino-($C_1$-$C_4$)-alkyl, $C_1$-$C_4$)-Alkoxycarbonylamino-($C_1$-$C_4$)-alkyl ($C_6$-$C_{12}$)-Aryl-($C_1$-$C_4$)-alkoxycarbonylamino-($C_1$-$C_4$)-alkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino und/oder Methylenoxy mono-, di- oder trisubstituiert sein kann, oder 3-Indolyl,
$R^1$ Wasserstoff oder ($C_2$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_3$ bis $C_9$)-Cycloalkyl, ($C_5$ bis $C_9$)-Cyclo-alkenyl, ($C_3$ bis $C_7$)-Cycloalkyl-($C_1$ bis $C_4$)-alkyl, ($C_6$ bis $C_{12}$)-Aryl- oder teilhydriertes Aryl, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_2$ bis $C_4$)-alkyl oder ($C_7$ bis $C_{13}$)-Aroyl-($C_1$-$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, geschützten α-Aminosäure und
$R_2$ und $R_3$ Wasserstoff, ($C_1$ bis $C_6$)-Alkyl, ($C_2$ bis $C_6$)-Alkenyl oder ($C_6$ - $C_{12}$)Aryl-($C_1$ bis $C_4$)-alkyl bedeuten.

Bevorzugt sind solche Verbindungen der Formel II und III, in welchen
n = 2 ist,
R Methyl, Cyclohexyl, tert. Butoxycarbonylamino-($C_1$-$C_4$) alkyl, Benzyloxycarbonylamino-($C_1$-$C_4$)alkyl oder Phenyl, das durch Phenyl ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkylamine, Nitro und/oder Methylendioxy mono- oder disubstituiert oder im Falle von Methoxy, trisubstituiert sein kann,
$R^1$ Wasserstoff, ($C_2$ oder $C_3$)-Alkyl, ($C_2$ oder $C_3$)-Alkenyl, die geschützte Seitenkette von Lysin, 4-Methoxybenzyl, 4-Ethoxybenzyl, Phenethyl, 4-Amino-butyl oder Benzoylmethyl
$R^2$ und $R^3$ Wasserstoff, ($C_1$ bis $C_4$)-Alkyl oder Benzyl bedeuten,
insbesondere aber Verbindungen der Formeln II und III, in welchen
n = 2 ist,
R Methyl, tert.Butoxycarbonylamino-ethyl, Benzyloxycarbonylamino-ethyl, Phenyl oder mit Fluor und/oder Chlor monooder disubstituiertes Phenyl, $R^1$ Ethyl, Phenyl, die acylierte Seitenkette von Lysin oder die O-($C_1$-$C_6$) -alkylierte Seitenkette von Tyrosin,
$R^2$ und $R^3$ Wasserstoff, Methyl, Ethyl tert. Butyl oder Benzyl bedeuten.

Die Trifluormethansulfonate der Formeln II und III erhält man, indem man α-Hydroxycarbonsäurederivate der Formeln VI bzw. VII

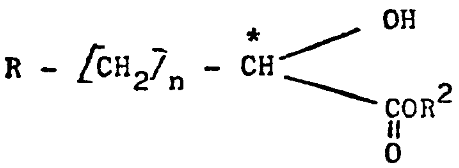

(VI)

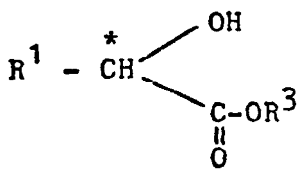

(VII)

in welchen n, R, $R^1$, $R^2$ und $R^3$ die oben genannten Bedeutungen haben, mit einem Trifluormethansulfonierungsmittel, wie z.B. Trifluormethansulfonsäureanhydrid oder Trifluormethansulfonylchlorid in einem inerten Lösungsmittel umsetzt.

Um die bei der Reaktion entstehende Säure abzufangen, ist es vorteilhaft, die Reaktion in Gegenwart einer Base durchzuführen. Dafür eignen sich anorganische Salze wie Carbonate (z.B. $K_2CO_3$, $Na_2CO_3$, $NaHCO_3$), $Na_2SO_4$ oder organische Basen wie z.B. Triethylamin, Pyridin. Man kann die Base in stöchiometrischer Menge oder auch im Überschuß verwenden.

Als Lösungsmittel sind diejenigen geeignet, die nicht mit dem Trifluormethansulfonierungsmittel und den Trifluormethansulfonsäurederivaten reagieren können. Dies sind z.B. Lösungsmittel wie Methylenchlorid, Chloroform, Tetrachlorkohlenstoff, oder andere halogenierte Kohlenwasserstoffe oder auch Kohlenwasserstoffe wie z.B. Hexan. Die Reaktion kann in einem Temperaturbereich zwischen -80° C und +80° C ausgeführt werden. Besonders vorteilhaft ist die Reaktion in Methylenchlorid, Chloroform oder Tetrachlorkohlenstoff, wobei Trifluormethansulfonsäureanhydrid in Gegenwart von Pyridin mit dem α-Hydroxycarbonsäurederivat bei Temperaturen zwischen -80°C und Raumtemperatur umgesetzt wird. Trifluormethansulfonsäureanhydrid kann auch im Überschuß verwendet werden.

Werden optisch aktive Verbindungen der Formeln VI oder VII eingesetzt, so bleibt bei der Überführung in die Verbindungen der Formeln II oder III die Konfiguration am chiralen Kohlenstoffatom erhalten.

Die Trifluormethansulfonsäurederivate der Formeln II bzw. III reagieren mit Aminosäureestern der Formeln IV bzw. V glatt zu Verbindungen der Formel I. Um die entstehende Trifluormethansulfonsäure abzufangen, wird die Reaktion zweckmäßig in Gegenwart eines Äquivalents einer Base durchgeführt, die nicht mit Verbindungen der Formeln II oder III abreagieren kann. Als vorteilhaft haben sich tert. Amine wie Triethylamin oder Pyridin erwiesen. Auch die Aminosäurederivate selbst können als Säurefänger dienen. Geeignet sind auch anorganische Salze wie z.B. $Na_2CO_3$, $K_2CO_3$, $NaHCO_3$, $Na_2SO_4$.

Die Reaktion wird in einem aprotischen polaren Lösungsmittel oder unpolaren Lösungsmittel durchgeführt. Geeignete Lösungsmittel sind z.B. Methylenchlorid, Chloroform, Tetrachlorkohlenstoff Dimethylformamid Essigsäureethylester, Dimethoxyethan, Hexan, Ether, Tetrahydrofuran.

Die Reaktionstemperatur liegt im Bereich zwischen -80 und + 150°C. Besonders vorteilhaft hat sich -20 bis +80°C erwiesen.

Die Aufarbeitung gestaltet sich sehr einfach. Das Lösungsmittel wird mit Wasser gewaschen, um die gebildeten Salze zu entfernen. Die organische Lösung wird nach dem Trocknen eingeengt, wobei die Verbindungen der Formeln I rein anfallen, die erforderlichenfalls nach den allgemeinen Reinigungsmethoden, wie z.B. u.a. Filtration oder Chromatographie über Kieselgel, hoch gereinigt werden können.

Werden optisch reine Verbindungen der Formeln II oder III in die Reaktion eingesetzt, so erfolgt die Substitution des Trifluormethansulfonsäureesters durch die Aminosäurederivate der Formel IV oder V unter Konfigurationsumkehr. Man erhält ohne Racemisierung aus optisch reinen Ausgangsprodukten optisch reine Endprodukte. Ein Diastereomerengemisch erhält man, wenn z.B. racemische Verbindungen der Formel II oder mit optisch reinen Aminosäurederivaten oder umgekehrt, oder racemische Verbindungen der Formel II oder III mit racemischen Aminosäurederivaten umgesetzt werden. Die entstandenen Diastereomeren können nach den allgemein üblichen Trennmethoden, wie z.B. u.a. fraktionierte Kristallisation der Salze oder Säulenchromatographie über Kieselgel getrennt werden. Auch wenn eine der Ausgangskomponenten racemisch ist, bietet das erfindungsgemäße Verfahren aufgrund der hohen Ausbeuten und Reinheit große Vorteile gegenüber bekannten Verfahren.

Die Verbindungen der Formeln I, II und III sind wertvolle Zwischenprodukte bei der Herstellung von Verbindungen der Formel VIII

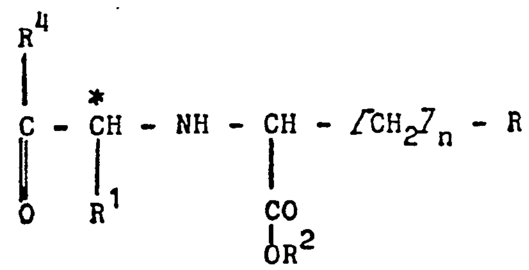

(VIII)

in welcher n, R, $R^1$ und $R^2$ die oben genannten Bedeutungen besitzt und $R^4$ für den über das Imino-Stickstoffatom verknüpften Rest einer mono-, bi- oder tricyclischen Imino-carbonsäure steht. Verbindungen der Formel VIII sind beispielsweise bekannt aus US-PS 4 350 704,US-PS 4 344 949, US-PS 4 374 847, EP-A 50 800, EP-A 31 741, EP-A 51 020, EP-A 49 658, EP-A 49 605, EP-A 29 488, EP-A 46 953 und EP-A 52 870. Sie sind weiterhin Gegenstand der deutschen Patentanmeldungen P 32 26 768.1, P 31 51 690.4, P 32 10 496.0, P 32 11 397.8, P 32 11 676.4, P 32 27 055.0, P 32 42 151.6, P 32 46 503. und P 32 46 757.5.

Die Verbindungen der Formel VIII sind Hemmer des Angiotensin-Converting-Enzyms (ACE) und können zur Bekämpfung des Bluthochdrucks verschiedener Genese eingesetzt werden.

Verbindungen der Formel VIII werden erhalten, indem man die entsprechenden α-Iminocarbonsäuren $R^4$-H oder deren Derivate mit Verbindungen der Formel I nach bekannten Amidbildungsmethoden der Peptidchemie umsetzt.

Die nachfolgenden Beispiele sollen das erfindungsgemäße Verfahren erläutern, ohne die Erfindung auf die hier stellvertretend genannten Substanzen zu beschränken.

**Beispiel I:**

N-(1-S-Carbethoxy-3-phenyl-propyl)-S-alaninbenzylester

a) 2-R,S-Trifluormethansulfonyloxy-4-phenylbuttersäureethyl-ester

Zu einer Lösung von 6.24 g (30 mMol) 2-R,S-Hydroxy-4-phenylbuttersäureethylester in 30 ml trockenem Methylenchlorid tropft man unter Rühren bei 0°C innerhalb einer Stunde eine Lösung von 2.37 g (30 mMol) trockenem Pyridin und 9.73 g (34.5 mMol) Trifluormethansulfonsäureanhydrid in 8 ml trockenem Methylenchlorid zu. Nach dem Zutropfen wird 15 min. bei 0°C nachgerührt. Es wird vom Niederschlag abgesaugt und die Methylenchloridlösung zweimal mit Wasser gewaschen. Nach dem Trocknen über $MgSO_4$ wird die Methylenchloridlösung eingeengt.

Ausbeute: 8.6 g = 84,3 % d.Th.

$R_f$: 0.37 ($SiO_2$; Cyclohexan / Essigsäureäthylester 4: 1; Molybdatophosphorsäure 15 % in Methanol)

b) N-(1-S-Carbethoxy-3-phenylpropyl)-S-alaninbenzylester

4.4 g (24.68 mMol) S-Alaninbenzylester und 2.5 g (24.7 mMol) Triäthylamin werden in 20 ml trockenem Methylenchlorid gelöst. Zu dieser Lösung werden unter Rühren und bei Raumtemperatur 8.4 g 2-R,S-Trifluormethansulfonyloxy-4-phenylbuttersäureethylester aus Beispiel Ia in 10 ml trockenem Methylenchlorid zugetropft. Es wird 40 min bei Raumtemperatur nachgerührt. Die Methylenchloridlösung wird drei Mal mit Wasser gewaschen, über $Na_2SO_4$ getrocknet und im Vakuum eingeengt.

Ausbeute: 9.0 g (99 % d.Th.) N-(1-R,S-Carbethoxy-3-phenylpropyl)-S-alaninbenzylester-Diastereomerengemisch

$R_f$ des Diastereomeren I: 0.12

$R_f$ des Diastereomeren II: 0.07

($SiO_2$; Cyclohexan / Essigsäureethylester 9:1, Molybdatophosphorsäure 15 % in Methanol)

Die beiden Diastereomeren lassen sich leicht über Kieselgel mit dem Laufmittelgemisch Cyclohexan / Essigsäureethylester 9:1 trennen.

Das langsam laufende Isomere besitzt die S,S-Konfiguration.

**Beispiel II:**

N-(1-S-Carbethoxy-3-phenylpropyl)-S-alaninbenzylester

a) 2-R-Trifluormethansulfonyloxy-4-phenylbuttersäureethyl-ester

Die Verbindung wird analog der Herstellvorschrift des Beispiel Ia aus 2-R-Hydroxy-4-phenylbuttersäureethylester und Trifluormethansulfonsäureanhydrid erhalten. Der Ethylester wird aus 2-R-Hyxdroxy-4-phenylbuttersäure (Biquard, Annales de Chimie 20, S. 145 (1933)) und absolutem Ethanol durch Einleiten von trockenem Chlorwasserstoffgas in die auf dem Wasserbad erwärmte Lösung, analog der Vorschrift Biquard, Annales de Chimie 20, 147 (1933), hergestellt.

$R_f$: 0.11 ($SiO_2$; Cyclohexan / Essigsäureäthylester 9:1).

Ausbeute: 90 % d.Th.

b) N-(1-S-Carbethoxy-3-phenylpropyl)-S-alaninbenzylester

Analog Beispiel Ib wird 2-R-Triflucrmethansulfonyloxy-4-phenylbuttersäureäthylester mit S-Alaninbenzylester umgesetzt. Unter Konfigurationsumkehr im Buttersäureteil wird die gewünschte S,S-Verbindung in 92 % Ausbeute erhalten.

$R_f$: 0.07 ($SiO_2$, Cyclohexan / Essigsäureethylester 9:1).

Die Konfiguration wurde auf folgende Weise bestimmt. Werden die in Beispiel I b (Diastereomer II) und Beispiel II b erhaltenen Verbindungen in Ethanol mit 10 % Palladium auf Kohle hydriert, so erhält man jeweils N-(1-S-Carbethoxy-3-phenylpropyl)-S-alanin mit einem optischen Drehwert $[\alpha]^{22}_D = +28°$ (c = 1, $CH_3OH$). Dieser Wert stimmt mit dem Literaturwert (EP-A 37 231, S. 30) von +31° (c = 1, $CH_3OH$) gut überein. Das 270 MHz $^1$H-NMR zeigt keine Verunreinigung durch das R,S-Diastereomere.

**Beispiel III**

N-(1-R,S-Carbethoxy-3-phenylpropyl)-O-ethyl-S-tyrosinbenzyl-ester

6.1 g 2-R,S-Trifluormethansulfonyloxy-4-phenylbuttersäure-ethylester aus Beispiel Ia werden mit 5.4 g O-Ethyl-Styrosinbenzylester und 1.8 g Triethylamin in Methylenchlorid analog Beispiel Ib umgesetzt.

Ausbeute: 95 % d.Th. 1:1 Diastereomerengemisch aus S,S und R,S-Verbindung.

$R_f$ Diastereomer I: 0.46 ($SiO_2$; Cyclohexan / Diisoproylether 1:1)

$R_f$ Diastereomer II: 0.39 ($SiO_2$; Cyclohexan / Diisopropylether 1:1)

Beide Diastereomere lassen sich leicht über Kieselgel mit Cyclohexan / Diisoproylether 8:2 trennen. Das langsam laufende Diastereomer besitzt die S,S-Konfiguration.

**Beispiel IV**

N-(1-S-Carbethoxy-3-phenylpropyl)-O-ethyl-S-tyrosinbenzyl-ester

Die Verbindung wird erhalten durch Umsetzung von 2-R-Tri-fluormethansulfonyloxy-4-phenylbuttersäureethylester aus Beispiel IIa, O-Ethyl-S-tyrosinbenzylester und Triethylamin im trockenen Methylenchlorid analog Beispiel Ib. Ausbeute: 95 % d.Th.

$R_f$: 0.39 ($SiO_2$; Cyclohexan / Diisopropylether, 1:1)

**Beispiel V**

N-(1-R,S-Carbethoxy-3-phenylpropyl)-O-methyl-S-tyrosinbenzyl-ester

Das Diastereomerengemisch wird erhalten durch Umsetzung von 2-R,S-Trifluormethansulfonyloxy-4-phenylbuttersäureethylester aus Beispiel Ia mit O-methyl-S-tyrosinbenzylester und Triethylamin in Methylenchlorid analog Beispiel Ib. Ausbeute: 92 % d.Th. Diastereomerengemisch

$R_f$ Diastereomer I: 0.23 ($SiO_2$; Cyclohexan / Essigsäureethylester 9:1).

$R_f$ Diastereomer II: 0.19 ($SiO_2$; Cyclohexan / Essigsäureethylester 9:1).

Die Diastereomere lassen sich leicht über Kieselgel mit Cyclohexan / Diisopropylether trennen. Das langsam laufende Isomer besitzt die S,S-Konfiguration.

**Beispiel VI**

N-(1-S-Carbethoxy-3-phenylpropyl)-O-methyl-S-tyrosinbenzyl-ester

Die Verbindung wird erhalten durch Umsetzung von 2-R-Tri-fluormethansulfonyloxy-4-phenylbuttersäureethylester aus Beispiel IIa mit O-Methyl-S-tyrosinbenzylester und Triethylamin in Methylenchlorid analog Beispiel Ib.

Ausbeute: 94 % d.Th.

$R_f$: 0.19 ($SiO_2$; Cyclohexan / Essigsäureethylester 9:1).

**Beispiel VII**

N (1 R S Carbethoxy 3 phenylpropyl) Nε -benzyloxycarbonyl-S-lysin-tert.butylester
Das Diastereomerengemisch wird erhalten durch Umsetzung von 2-R,S-Trifluormethansulfonyloxy-4-phenylbuttersäureethylester aus Beispiel Ia mit $N_ε$ -Benzyloxycarbonyl-S-lysin-tert. Butylester und Triethylamin in Methylenchlorid analog Beispiel Ib.
Ausbeute: 95 % d.Th.
m/e: 526

**Beispiel VIII**

$N_α$-(1-S-Carbethoxy-3-phenylpropyl)-$N_ε$-benzyloxycarbonyl-S-lysin-tert.butylester
Die Verbindung wird erhalten durch Umsetzung von 2-R-Tri-fluormethansulfonyloxy-4-phenylbuttersäureethylester aus Beispiel IIa mit Nε -Benzyloxycarbonyl-S-lysin-tert.Butylester und Triethylamin in Methylenchlorid analog Beispiel Ib. Ausbeute 82 % d.Th.
m/e: 526

**Beispiel IX**

N-(1-R,S-Carbethoxy-3-phenyl-propyl)-S-alaninbenzylester
a) 2-R-Trifluormethansulfonyloxy-propionsäurebenzylester
Die Verbindung wird erhalten durch Umsetzung von D-Milchsäurebenzylester mit Trifluormethansulfonsäureanhydrid und Pyridin im Methylenchlorid analog Beispiel Ia.
Ausbeute: 95 % d.Th.
b) N-(1-R,S-Carbethoxy-3-phenyl-propyl)-S-alaninbenzylester
Man erhält das Diastereomerengemisch durch Umsetzung von 2-R-Trifluormethansulfonyloxy-propionsäurebenzylester mit R,S-Homophenylalaninäthylester und Triethylamin in Methylenchlorid analog Beispiel Ib.
Ausbeute 90 % d.Th.
$R_f$ Diastereomer I: 0.12 ($SiO_2$; Cyclohexan / Essigsäureethylester 9:1).
$R_f$ Diastereomer II: 0.07 ($SiO_2$; Cyclohexan / Essigsäureethylester 9:1).
Die physikalischen Daten stimmen mit denen der Diastereomeren aus Beispiel Ib überein.

**Beispiel X**

N-(1-S-Carbethoxy-3-phenylpropyl)-S-alaninbenzylester
Man erhält die Verbindung durch Umsetzung von 2-R-Trifluor-methansulfonyloxy-propionsäurebenzylester mit S-Homophenylalaninethylester und Triethylamin in Tetrachlorkohlenstoff analog Beispiel Ib.
Ausbeute: 98 % d.Th.
$R_f$: 0.07 ($SiO_2$; Cyclohexan, Essigsäureethylester 9:1).
Die physikalischen Daten stimmen mit denen der Verbindung aus Beispiel IIb überein.

**Beispiel XI**

$N_α$ -(1-S-Carbobenzyloxy-ethyl)-$N_ε$ -tert.butoxycarbonyl-Slysinethylester
Die Verbindung wird erhalten durch Umsetzung von 2-R-Tri-fluormethansulfonyloxypropionsäurebenzylester aus Beispiel IX a mit $N_ε$ -tert. Butoxycarbonyl-S-lysinethylester und Triethylamin im Methylenchlorid analog Beispiel Ib. Ausbeute: 82 % d.Th.
m/e: 436

**Beispiel XII**

N-(1-R,S-Carbethoxy-3-phenylpropyl)-R-alaninbenzylester
Das Diastereomerengemisch wird erhalten durch Umsetzung von 2-R,S-Trifluormethansulfonyloxy-buttersäurebenzylester mit R-Alaninbenzylester und Triethylamin im Methylenchlorid analog Beispiel Ib und

durch Umsetzung von 2-S-Trifluor-methansulfonyloxy-milchsäurebenzylester und R,S-Homophenylalaninethylester analog Beispiel Ib.

Ausbeute: 92 % d.Th.

$R_f$ Diastereomer I: 0.13 ($SiO_2$; Cyclohexan / Essigsäureethylester 9:1)

$R_f$ Diastereomer II: 0.07 ($SiO_2$; Cyclohexan / Essigsäureethylester 9:1)

**Beispiel XIII**

N-(1-S-Carbethoxy-3-phenylpropyl)-R-alaninbenzylester

Die Verbindung wird erhalten durch Umsetzung von 2-S-Tri-fluormethansulfonyloxypropionsäurebenzylester, hergestellt aus L-Milchsäurebenzylester, Trifluormethansulfonsäureanhydrid und Pyridin in Methylenchlorid analog Beispiel Ia, mit S-Homophenylalaninethylester und Triethylamin in Methylenchlorid analog Beispiel Ib.

Ausbeute: 95 % d.Th.

$R_f$: 0.08 ($SiO_2$; Cyclohexan / Essigsäureethylester 9:1)

**Beispiel XIV**

$N_\alpha$-(1-S-Carb.-tert.butoxy-ethyl)-$N_\varepsilon$-benzyloxycarbonyl-S-lysinethylester

Die Verbindung wird erhalten durch Umsetzung von 2-R-Tri-fluormethansulfonyloxy-propionsäure-tert.butylester und Nε-Benzyloxycarbonyl-S-lysinethylester und Triethylamin in Methylenchlorid analog Beispiel Ib.

Nach den oben angegebenen Verfahren werden ausgehend von den entsprechenden 2-Hydroxycarbonsäureestern, die in der R bzw. S oder R,S-Form eingesetzt werden (bevorzugt werden die R und R,S-Formen), die folgenden Triflate hergestellt:

2-R,S-Trifluormethansulfonyloxy-4-(4-fluorphenyl)-buttersäureethylester
2-R-Trifluormethansulfonyloxy-4-(4-fluorphenyl)-buttersäureethylester
2-R,S-Trifluormethansulfonyloxy-4-(4-methoxyphenyl)-buttersäureethylester
2-R-Trifluormethansulfonyloxy-4-(4-methoxyphenyl)-buttersäureethylester
2-R,S-Trifluormethansulfonyloxy-4-(4-chlorphenyl)-buttersäureethylester
2-R-Trifluormethansulfonyloxy-4-(4-chlorphenyl)-buttersäureethylester
2-R,S-Trifluormethansulfonyloxy-4-(3,4-dichlorphenyl)-buttersäureethylester
2-R-TriTluormethansulfonyloxy-4-(3,4-dichlorphenyl)-buttersäureethylester
2-R,S-Trifluormethansulfonyloxy-4-(2-methylphenyl)-buttersäureethylester
2-R-Trifluormethansulfonyloxy-4-(2-methylphenyl)-buttersäureethylester
2-R,S-Trifluormethansulfonyloxy-4-(3,4-dimethoxyphenyl)-buttersäureethylester
2-R-Trifluormethansulfonyloxy-4-(3,4-dimethoxyphenyl)-buttersäureethylester
2-R,S-Trifluormethansulfonyloxy-4-(4-phenylphenyl)-buttersäureethylester
2-R-Trifluormethansulfonyloxy-4-(4-phenyphenyl)-buttersäureethylester
2-R,S-Trifluormethansulfonyloxy-buttersäureethylester
2-R-Trifluormethansulfonyloxy-buttersäureethylester
2-R,S-Trifluormethansulfonyloxy-4-cyclohexyl-buttersäureethylester
2-R-Trifluormethansulfonyloxy-4-cyclohexyl-buttersäureethylester
2-R,S-Trifluormethansulfonyloxy-3-(indol-3-yl)-propionsäureethylester
2-R-Trifluormethansulfonyloxy-3-(indol-3-yl)-propionsäureethylester
2-R,S-Trifluormethansulfonyloxy-3-(N-trifluormethansulfonyl-indol-3-yl)-propionsäureethylester
2-R-Trifluormethansulfonyloxy-3-(N-trifluormethansulfonyl-indol-3-yl)-propionsäureethylester

Die für die Herstellung der Triflate erforderlichen 2-R,S-Hydroxycarbonsäureester werden aus den entsprechenden α-Ketoestern durch Reduktion mit Raney-Nickel und Wasserstoff in Ethanol erhalten. Ein weiteres angewendetes Herstellungsverfahren besteht darin, daß die entsprechenden Cyanhydrine sauer verseift werden und die gebildeten 2-Hydroxycarbonsäuren in die Ethylester nach gängigen Veresterungsverfahren überführt werden.

Die Racematspaltung der racemischen 2-Hydroxycarbonsäuren geschieht entweder über diastereomere Salzbildung mit optisch aktiven Aminen oder Aminosäureester und fraktionierte Kristallisation oder durch Veresterung mit optisch aktiven Alkoholen wie z.B. Menthol, die säulenchromatographisch oder durch fraktionierte Kristallisation getrennt werden. Die Veresterung zu den optisch aktiven 2-Hydroxycarbonsäureestern geschieht nach den gängigen Veresterungsverfahren.

Nach dem in Beispiel Ib bzw. IIb angegebenen Verfahren werden die oben beschriebenen 2-Trifluormethansulfonyloxycarbonsäureester mit den entsprechenden Aminosäureestern analog zu den folgenden Verbindungen umgesetzt:

N-(1-S-Carbethoxy-3-[4-fluorphenyl] -propyl)-S-alaninbenzylester

N-(1-S-Carbethoxy-3-[4-methoxyphenyl] -propyl)-S-alaninbenzylester
N-(1-S-Carbethoxy-3-[4-chlorphenyl]-propyl)-S-alaninbenzylester
N-(1-S-Carbethoxy-3-[2-methylphenyl] -propyl)-S-alaninbenzylester
N-(1-S-Carbethoxy-3-[3,4-dimethoxyphenyl]-propyl)-S-alaninbenzylester
N-(1-S-Carbethoxy-butyl)-S-alaninbenzylester
N-(1-S-Carbethoxy-3- cyclohexyl -propyl)-S-alaninbenzylester
N-(1-S-Carbethoxy-3-/4-phenyl-phenyl/-propyl)-S-alaninbenzylester
N-(1-S-Carbethoxy-3-[4-fluorphenyl] -propyl)-O-methyl-Styrosinbenzylester
N-(1-S-Carbethoxy-3-[4-fluorphenyl]-propyl)-O-ethyl-S-tyrosinbenzylester
N-(1-S-Carbethoxy-3-[4-methoxyphenyl]-propyl)-O-ethyl-S-tyrosinbenzylester
N-(1-S-Carbethoxy-3-[4-methoxyphenyl]-propyl)-O-methyl-S-tyrosinbenzylester
N-(1-S-Carbethoxy-3-[2,6-dichlorphenyl]-propyl)-O-ethyl-S-tyrosinbenzylester
N-(1-S-Carbethoxy-3-[2,6-dichlorphenyl]-propyl)-O-methyl-S-tyrosinbenzylester
N-(1-S-Carbethoxy-3-[2,6-dichlorphenyl] -propyl)-S-alaninbenzylester
N-(1-S-Carbethoxy-3-[4-chlorphenyl]-propyl)-O-ethyl-S-tyrosinbenzylster
N-(1-S-Carbethoxy-3-[4-chlorpheny]-propyl)-O-methyl-S-tyrosinbenzylester
N-(1-Carbethoxy-3-[2-methylphenyl]-propyl)-O-ethyl-S-tyrosinbenzylester
N-(1-S-Carbethoxy-3-[2-methylphenyl]-propyl)-O-methyl-S-tyrosinbenzylester
N-(1-S-Carbethoxy-3-[3,4-dimethoxyphenyl]-propyl)-O-ethyl-tyrosinbenzylester
N-(1-S-Carbethoxy-3-[3,4-dimethoxyphenyl]-propyl)-O-methyl-S-tyrosinbenzylester
N-(1-S-Carbethoxy-butyl)-O-ethyl-S-tyrosinbenzylester
N-(1-S-Carbethoxy-butyl)-O-methyl-S-tyrosinbenzylester
N-(1-S-Carbethoxy-3- cyclohexyl -propyl)-O-ethyl-S-tyrosinbenzylester
N-(1-S-Carbethoxy-3- cyclohexyl -propyl)-O-methyl-S-tyrosinbenzylester
$N_\alpha$-(1-S-Carbethoxy-3-[4-methoxyphenyl]-propyl)-$N_\varepsilon$-tert. butoxycarbonyl-S-lysinbenzylester
$N_\alpha$-(1-S-Carbethoxy-3-[4-chlorphenyl]-propyl)-$N_\varepsilon$-benzyloxy-carbonyl-S-lysin-tert.butylester
$N_\alpha$-(1-S-Carbethoxy-3-[4-chlorphcnyl]-propyl)-$N_\varepsilon$-tert. butoxycarbonyl-S-lysinbenzylester
$N_\alpha$-(1-S-Carbethoxy-3-[2,6-dichlorphenyl]-propyl)-$N_\varepsilon$-tert. butoxycarbonyl-S-lysinbenzylester
$N_\alpha$-(1-S-Carbethoxy-3-[2.6-dichlorphenyl]-propyl)-$N_\varepsilon$-benzyl-oxycarbonyl-S-lysin-tert.butylester
$N_\alpha$(1-S-Carbethoxy-3-[2-methylphenyl]-propyl)-$N_\varepsilon$-benzyloxy-carbonyl-S-lysin-tert.butylester
$N_\alpha$-(1-S-Carbethoxy-3-[2-methylphenyl]-propyl)-$N_\varepsilon$-tert. butoxycarbonyl-S-lysinbenzylester
$N_\alpha$-(1-S-Carbethoxy-3-[3,4-dimethoxyphenyl]-propyl)-$N_\varepsilon$ -tert.butoxycarbonyl-S-lysinbenzylester
$N_\alpha$-(1-S-Carbethoxy-3-[3,4-dimethoxy-phenyl]-propyl)-$N_\varepsilon$ -benzyloxcarbonyl-S-lysin-tert.butylester
$N_\alpha$-(1-S-Carbethoxy-butyl)-$N_\varepsilon$-benzyloxycarbonyl-S-lysin-tert. butylester
$N_\alpha$-(1-S-Carbethoxy-butyl)-$N_\varepsilon$-tert.butoxycarbonyl-S-lysinbenzylester
$N_\alpha$-(1-S-Carbethoxy-3- cyclohexyl-propyl)-$N_\varepsilon$-tert.butoxycarbonyl-S-lysinbenzylester
$N_\alpha$-(1-S-Carbethoxy-3- cyclohexyl -propyl)-$N_\varepsilon$-benzyloxycarbonyl-S-lysin -tert.butylester
$N_\alpha$-(1-S-Carbobenzyloxy-ethyl)-$N_\varepsilon$-tert.butoxycarbonyl-S-lysin ethylester
$N_\alpha$-(1-S-Carbotert.butoxy-2-/4-ethoxyphenyl/-ethyl)-$N_\varepsilon$-benzyloxycarbonyl-S-lysin-ethylester
$N_\alpha$-(1-S-Carbotert.butoxy-2-[4-methoxyphenyl]-ethyl)-$N_\varepsilon$ -benzyloxycarbonyl-S-lysinethylester

Bei Anwendung der entsprechenden S-Aminosäure-tert.butyl ester-Edukte werden anstelle der Benzylesterprodukte die tert.Butylesterendprodukte erhalten.

Bei Anwendung der racemischen Triflat-Edukte, werden die entsprechenden S-Aminsäureester erhalten, die die R,S-Konfiguration im N-Alkylteil besitzen.

**Patentansprüche** für die Vertragsstaaten: BE, CH, DE, FR, GB, IT, LU, LI, NL, SE

1. Verfahren zur Herstellung von Verbindungen der Formel I

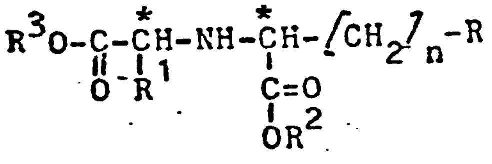

(I)

in welcher
n = 1 oder 2 ist,
R Wasserstoff,
Carboxy, Carbamoyl,

einen gegebenenfalls substituierten aliphatischen Rest mit 2-8 C-Atomen,

einen gegebenenfalls substituierten cycloaliphatischen Rest mit 3-9 C-Atomen, einen gegebenenfalls substituierten aromatischen Rest mit 8-12 C-Atomen,

einen Rest $OR^4$ oder $SR^4$, worin

$R^4$ für einen gegebenenfalls substituierten aliphatischen Rest mit 1-4 C-Atomen, für einen gegebenenfalls substituierten aromatischen Rest mit 8-12 C-Atomen oder einen gegebenenfalls substituierten heteroaromatischen Rest mit 5-10 Ringatomen steht, oder

einen gegebenenfalls substituierten heteroaromatischen Rest mit 5-10 Ringatomen,

$R^1$ Wasserstoff,

einen gegebenenfalls substituierten aliphatischen Rest mit 1-8 C-Atomen,

einen gegebenenfalls substituierten cycloaliphatischen Rest mit 3-9 C-Atomen,

einen gegebenenfalls substituierten cycloaliphatischaliphatischen Rest mit 4-13 C-Atomen,

einen gegebenenfalls substituierten aromatischen Rest mit 6-12 C-Atomen,

einen gegebenenfalls substituierten araliphatischen Rest mit 7-18 C-Atomen,

einen gegebenenfalls substituierten heteroaromatischen Rest mit 5-10 Ringatomen oder

die Seitenkette einer gegebenenfalls geschützten natürlich vorkommenden α-Aminosäure bedeuten,

$R^2$ und $R^3$ gleich oder verschieden sind und

Wasserstoff,

einen gegebenenfalls substituierten aliphatischen Rest mit 1-6 C-Atomen,

einen gegebenenfalls substituierten cycloaliphatischen Rest mit 3-9 C-Atomen,

einen gegebenenfalls substituierten cycloaliphatischaliphatischen Rest mit 4-12 C-Atomen,

einen gegebenenfalls substituierten aromatischen Rest mit 6-12 C-Atomen oder

einen gegebenenfalls substituierten araliphatischen Rest mit 7-16 C-Atomen

bedeuten,

dadurch gekennzeichnet, daß man Verbindungen der Formeln II bzw. III

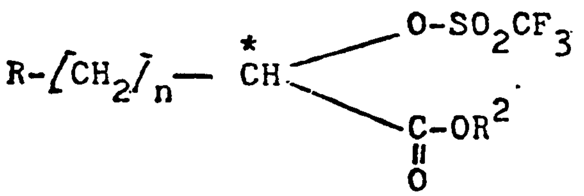

(II)

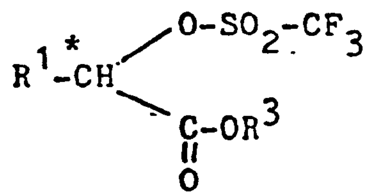

(III)

in welchen n, R, $R^1$, $R^2$ und $R^3$ die oben genannten Bedeutungen haben, mit Verbindungen der Formeln IV bzw. V

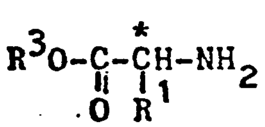

(IV)

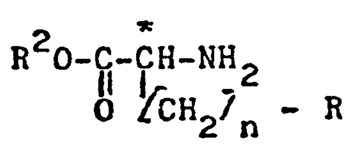

(V)

in denen R, $R^1$, $R^2$, $R^3$ und n die oben genannten Bedeutungen haben, umsetzt, gegebenenfalls Estergruppen hydrolytisch oder hydrogenolytisch abspaltet oder gegebenenfalls freie Carboxygruppen in an sich bekannter Weise verestert.

2. Verfahren gemäß Anspruch 1 dadurch gekennzeichnet,

daß

n = 1 oder 2 ist

R Wasserstoff,
Alkyl mit 2-8 C-Atomen,
Alkenyl mit 2-6 C-Atomen,
Cycloalkyl mit 3-9 C-Atomen,
Aryl mit 6-12 C-Atomen,
das durch $(C_1-C_4)$-Alkyl, $(C_1-C_4)$-Alkoxy, Hydroxy, Halogen, Nitro, Amino, Aminomethyl$(C_1-C_4)$-Alkylamino, Di-$(C_1-C_4)$-alkylamino, $(C_1-C_4)$-Acylamino, vorzugsweise $(C_1-C_4)$-Alkanoylamino, Methylendioxy, Carboxy, Cyano und/oder Sulfamoyl mono-, di- oder trisubstituiert sein kann,
Alkoxy mit 1-4 C-Atomen,
Aryloxy mit 6-12 C-Atomen,
das wie oben bei Aryl beschrieben substituiert sein kann,
mono bzw. bicyclisches Heteroaryloxy mit 5-7 bzw. 8-10 Ringatomen, wovon 1 bis 2 Ringatome Schwefeloder Sauerstoffatome und/oder 1 bis 4 Ringatome Stickstoff darstellen,
das wie oben bei Aryl beschrieben substituiert sein kann,
Amino-(C1-C4)-alkyl,
$(C_1-C_4)$-Alkanoylamino-$(C_1-C_4)$-alkyl,
$(C_7-C_{13})$-Aroylamino-$(C_1-C_4)$-alkyl,
$(C_1-C_4)$-Alkoxy-carbonylamino-$(C_1-C_4)$-alkyl,
$(C_6-C_{12})$-Aryl-$(C_1-C_4)$-alkoxy-carbonylamino-$(C_1-C_4)$-alkyl
$(C_6-C_{12})$-Aryl-$(C_1-C_4)$-alkylamino-$(C_1-C_4)$-alkyl,
$(C_1-C_4)$-Alkylamino-$(C_1-C_4)$-alkyl,
Di-$(C_1-C_4)$-alkylamino-$(C_1-C_4)$-alkyl,
Cuanidino-$(C_1-C_4)$-alkyl,
Imidazolyl, Indolyl,
$(C_1-C_4)$-Alkylthio,
$(C_1-C_4)$-Alkylthio-$(C_1-C_4)$-alkyl,
$(C_6-C_{12})$-Arylthio-$(C_1-C_4)$-alkyl,
das im Arylteil wie oben bei Aryl beschrieben, substituiert sein kann,
$(C_6-C_{12})$-Aryl-$(C_1-C_4)$-alkylthio,
das im Arylteil wie oben bei Aryl beschrieben, substituiert sein kann
Carboxy-$(C_1-C_4)$-alkyl,
Carboxy, Carbamoyl,
Carbamoyl-$(C_1-C_4)$-alkyl,
$(C_1-C_4)$-Alkoxy-carbonyl-$(C_1-C_4)$-alkyl,
$(C_1-C_4)$Alkoxycarbonyl
$(C_6-C_{12})$-Aryloxy-$(C_1-C_4)$-alkyl,
das im Arylteil wie oben bei Aryl beschrieben substituiert sein kann oder
$(C_6-C_{12})$-Aryl-$(C_1-C_4)$-alkoxy,
das im Arylteil wie oben bei Aryl beschrieben substituiert sein kann,
$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6-12 C-Atomen, das wie oben bei R beschrieben substituiert sein kann,$(C_6-C_{12})$-Aryl-$(C_1-C_4)$-alkyl oder $(C_7-C_{13})$-Aroyl-($C_1$ oder $C_2$)alkyl
die beide wie das vorstehende Aryl substituiert sein können,
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die Seitenkette einer gegebenenfalls geschützten natürlich vorkommenden α-Aminosäure $R_1$-CH($NH_2$)-COOH bedeuten,
$R^2$ und $R^3$ gleich oder verschieden sind und Wasserstoff, Alkyl mit 1-6 C-Atomen,
Alkenyl mit 2-6 C-Atomen,
Di-$(C_1-C_4)$-alkylamino-$(C_1-C_4)$-alkyl,
$(C_1-C_5)$-Alkanoyloxy-$(C_1-C_4)$-alkyl,
$(C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy$(C_1-C_4)$-alkyl
Aryl mit 6-12 C-Atomen,
$(C_6-C_{12})$ Aryl-$(C_1-C_4)$-alkyl,
$(C_3-C_9)$ Cycloalkyl oder

$(C_3$-$C_9)$-Cycloalkyl-$(C_1$-$C_4)$-alkyl bedeuten.

3. Verfahren gemäß Anspruch 1, dadurch gekennzeichnet, daß
n = 1 oder 2 ist,
R $(C_2$ bis $C_6)$-Alkyl $(C_2$ bis $C_6)$-Alkenyl, $(C_3$ bis $C_9)$-Cycloalkyl, Amino-$(C_1$-$C_4)$-alkyl, $(C_2$-$C_5)$-Acylamino-$(C_1$-$C_4)$-alkyl, $(C_1$-$C_4)$-Alkoxycarbonylamino-$(C_1$-$C_4)$alkyl, $(C_6$-$C_{12})$-Aryl-$(C_1$-$C_4)$-alkoxycarbonylamino-$(C_1$-$C_4)$-alkyl, $(C_7$-$C_{13})$-Aroylamino-$(C_1$-$C_4)$-alkyl, $(C_6$ bis $C_{12})$-Aryl, das durch $(C_1$ bis $C_4)$-Alkyl, $(C_1$ bis $C_4)$-Alkoxy, Hydroxy, Halogen, Nitro, Amino, $(C_1$ bis $C_4)$-Alkylamino, Di-$(C_1$ bis $C_4)$alkylamino und/oder Methylendioxy mono-, di- oder trisubstituiert sein kann, oder 3-Indolyl,
$R^1$ Wasserstoff oder $(C_1$ bis $C_6)$-Alkyl, das gegebenenfalls durch Amino, $(C_1$ bis $C_6)$-Acylamino oder Denzoylamino substituiert sein kann, $(C_2$ bis $C_6)$-Alkenyl, $(C_3$ bis $C_9)$-Cycloalkyl, $(C_5$ bis $C_9)$-Cycloalkenyl, $(C_3$ bis $C_7)$-Cycloalkyl-$(C_1$ bis $C_4)$-alkyl, $(C_6$ bis $C_{12})$-Aryl oder teilhydriertes Aryl, das jeweils durch $(C_1$ bis $C_4)$-Alkyl, $(C_1$ oder $C_2)$-Alkoxy oder Halogen substituiert sein kann, $(C_6$-$C_{12})$-Aryl-$(C_1$ bis $C_4)$-alkyl oder $(C_7$-$C_{13})$-Aroyl-$(C_1$-$C_2)$alkyl die beide wie vorstehend definiert im Arylrest substituiert sein können, ein mono- bzw. bicyclischer Hetcrocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure.
$R^2$ und $R^3$ gleiche oder verschiedene Reste Wasserstoff, $(C_1$ bis $C_6)$-Alkyl, $(C_2$ bis $C_6)$-Alkenyl oder $(C_6$ - $C_{12})$-Aryl-$(C_1$ bis $C_4)$-alkyl,
bedeuten.

4. Verfahren gemäß Anspruch 1, dadurch gekennzeichnet, daß
n = 1 oder 2 ist, $(C_1$-$C_4)$-Alkoxycarbonylamino-$(C_1$-$C_4)$-$(C_6$-$C_{12})$ Aryl $(C_1$-$C_4)$-alkoxycarbonylamino $(C_1$-$C_4)$-alkyl, Cyclohexyl oder Phenyl, das durch Phenyl, $(C_1$ oder $C_2)$-Alkyl, $(C_1$ oder $C_2)$-Alkoxy, Hydroxy, Fluor, Chlor, Brom, Amino, $(C_1$ bis $C_4)$-Alkylamino, Di-$(C_1$ bis $C_4)$alkyl-amino, Nitro und/ oder Methylendioxy mono- oder disubstituiert oder im Falle von Methoxy, trisubstituiert sein kann,
$R^1$ Wasserstoff, $(C_1$ bis $C_3)$-Alkyl, $(C_2$ oder $C_3)$-Alkenyl, die gegebenenfalls geschützte Seitenkette von Lysin, Benzyl, 4-Methoxybenzyl, 4-Ethoxy-benzyl, Phenethyl, 4-Amino-butyl oder Benzoylmethyl- und
R und $R^3$ gleiche oder verschiedene Reste Wasserstoff, $(C_1$ bis $C_4)$-Alkyl oder Benzyl
bedeuten.

5. Verbindung der Formel II

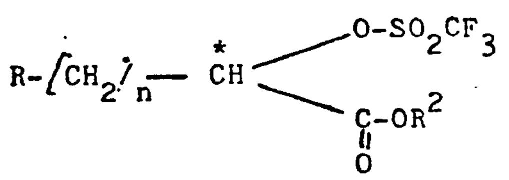

(II)

in welcher
n = 2 ist,
R $(C_1$ bis $C_6)$-Alkyl $(C_2$ bis $C_6)$-Alkenyl $(C_3$ bis $C_9)$-Cycloalkyl, $(C_2$-$C_5)$-Acylamino-$(C_1$-$C_4)$-alkyl, $(C_7$-$C_3)$-Aroylamino-$(C_1$-$C_4)$-alkyl, $(C_1$-$C_4)$-Alkoxycarbonylamino-$(C_1$-$C_4)$-alkyl, $(C_6$-$C_{12})$-Aryl-$(C_1$-$C_4)$-alkoxy,arbonylamino-$(C_1$-$C_4)$-alkyl, $(C_6$ bis $C_{12})$-Aryl, das durch $(C_1$ bis $C_4)$-Alkyl, $(C_1$ bis $C_4)$-Alkoxy-Hydroxy, Halogen, Nitro, Amino, $(C_1$ bis $C_4)$-Alkylamino, Di-$(C_1$ bis $C_4)$alkyl-amino und/oder Methylendioxy mono-, di- oder trisubstituiert sein kann, oder 3-Indolyl und
$R^2$ Wasserstoff, $(C_1$bis $C_6)$-Alkyl, $(C_2$ bis $C_6)$-Alkenyl oder $(C_6$ bis $C_{12})$-Aryl- (C bis $C_4)$-alkyl
bedeuten.

6. Verbindung der Formel III

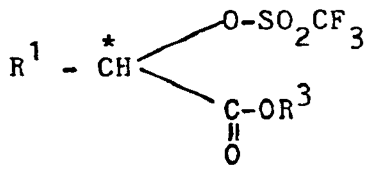

(III)

in welcher

$R^1$ Wasserstoff oder ($C_2$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_3$ bis $C_9$)-Cycloalkyl, ($C_5$ bis $C_9$)-Cycloalkenyl, ($C_3$ bis $C_7$)-Cycloalkyl-($C_1$ bis $C_4$)-alkyl, ($C_6$ bis $C_{12}$)-Aryl oder teilhydriertes Aryl, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_2$ bis $C_4$)-alkyl oder ($C_7$ - $C_{13}$)-Aroyl-($C_1$-$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer datürlich vorkommenden, geschützten α-Aminosäure und

$R^3$ Wasserstoff, ($C_1$ bis $C_6$)-Alkyl, ($C_2$ bis $C_6$)-Alkenyl oder ($C_6$ - $C_{12}$)-Aryl-($C_1$ bis $C_4$)-alkyl bedeuten.

7. Verbindung gemäß Anspruch 5, dadurch gekennzeichnet, daß R Ethyl, tert.Butoxycarbonylamino-($C_1$-$C_4$)-alkyl, Benzyloxycarbonylamino-($C_1$-$C_4$)-alkyl, Cyclohexyl oder Phenyl, das durch Phenyl, ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-(C bis $C_4$)-alkylamimo, Nitio und/oder Methylendioxy mono- oder disubstituiert oder im Falle von Methoxy, trisubstituiert sein kann, und

$R^2$ Wasserstoff, ($C_1$ bis $C_4$)-Alkyl oder Benzyl bedeuten.

8. Verbindung gemäß Anspruch 6, dadurch gekennzeichnet, daß

$R^1$ Wasserstoff, ($C_2$ oder $C_3$)-Alkyl, ($C_2$ oder $C_3$)-Alkenyl, die geschützte Seitenkette von Lysin, 4-Methoxybenzyl, 4-Ethoxybenzyl, Phenethyl, 4-Amino-butyl oder Benzoylmethyl und

$R^3$ Wasserstoff, ($C_1$ bis $C_4$)-Alkyl oder Benzyl

bedeuten.

9. Verbindung gemäß Anspruch 5, dadurch gekennzeichnet, daß

R Phenyl oder mit Fluor und/oder Chlor mono- oder disubstituiertes Phenyl und

$R^2$ Wasserstoff, Methyl, Ethyl, tert.Butyl oder Benzyl

bedeuten.

10. Verbindung gemäß Anspruch 6, dadurch gekennzeichnet, daß

$R^1$ die acylierte Seitenkette von Lysin oder die O-($C_1$ - $C_6$)-alkylierte Seitenkette von Tyrosin und

$R^3$ Wasserstoff, Methyl, Ethyl, tert. Butyl oder Benzyl

bedeuten.

11. Verfahren zur Herstellung von Verbindungen der Formeln II bzw. III, dadurch-gekennzeichnet, daß man α-Hydroxycarbonsäurederivate der Formeln VI bzw. VII

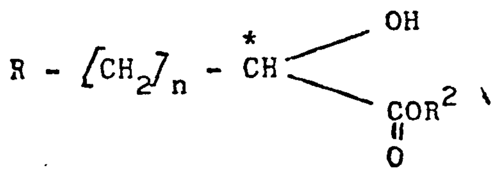

(VI)

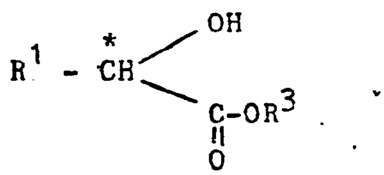

(VII),

in welchen n, R, $R^1$, $R^2$ und $R^3$ die oben genannten Bedeutungen haben, mit einem Trifluormethansulfonierungsmittel gegebenenfalls in Gegenwart einer Base umsetzt.

**Patentansprüche** für den Vertragsstaat: AT

1. Verfahren zur Herstellung von Verbindungen der Formel I

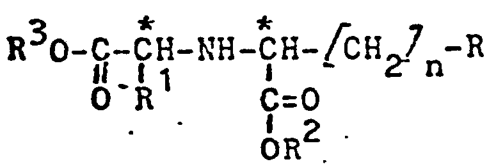

(I)

in welcher
n = 1 oder 2 ist,
R Wasserstoff,
Carboxy, Carbamoyl,
einen gegebenenfalls substituierten aliphatischen Rest mit 2-8 C-Atomen,
einen gegebenenfalls substituierten cycloaliphatischem Rest mit 3-9 C-Atomen, einen gegebenenfalls substituierten aromatischen Rest mit 6-12 C-Atomen,
einen Rest $OR^4$ oder $SR^4$, worin
$R^4$ für einen gegebenenfalls substituierten aliphatischen Rest mit 1-4 C-Atomen, für einen gegebcnenfalls substituierten aromatischen Rest mit 6-12 C-Atomen oder einen gegebenenfalls substituierten heteroaromatischen Rest mit 5-10 Ringatomen steht, oder
einen gegebenenfalls substituierten heteroaromatischen Rest mit 5-10 Ringatomen,
$R^1$ Wasserstoff,
einen gegebenenfalls substituierten aliphatischen Rest mit 1-6 C-Atomen,
einen gegebenenfalls substituierten cycloaliphatischen Rest mit 3-9 C-Atomen,
einen gegebenenfalls substituierten cycloaliphatischaliphatischen Rest mit 4-13 C-Atomen,
einen gegebenenfalls substituierten aromatischen Rest mit 6-12 C-Atomen,
einen gegebenenfalls substituierten araliphatischen Rest mit 7-16 C-Atomen,
einen gegebenenfalls substituierten heteroaromatischen Rest mit 5-10 Ringatomen oder
die Seitenkette einer gegebenenfalls geschützten natürlich vorkommenden α-Aminosäure bedeuten,
$R^2$ und $R^3$ gleich oder verschieden sind und Wasserstoff,
einen gegebenenfalls substituierten aliphatischen Rest mit 1-6 C-Atomen,
einen gegebenenfalls substituierten cycloaliphatischen Rest mit 3-9 C-Atomen,
einen gegebenenfalls substituierten cycloaliphatischaliphatischen Rest mit 4-12 C-Atomen,
einen gegebenenfalls substituierten aromatischen Rest mit 6-12 C-Atomen oder

einen gegebenenfalls substituierten araliphatischen Rest mit 7-16 C-Atomen bedeuten,
dadurch gekennzeichnet, daß man Verbindungen der Formeln II bzw. III

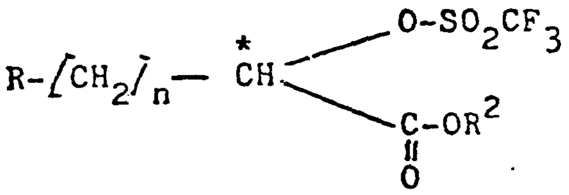

(II)

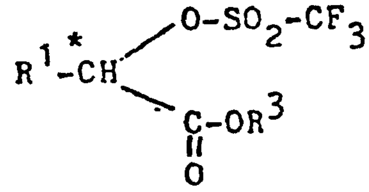

(III)

in welchen n, R, $R^1$, $R^2$ und $R^3$ die oben Bedeutungen haben, mit Verbindungen der Formeln IV bzw. V

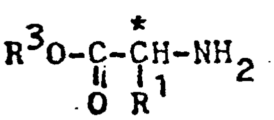

(IV)

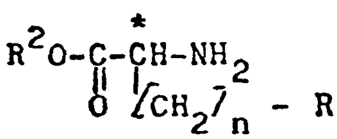

(V)

in denen R, $R^1$, $R^2$, $R^3$ und n die oben genannten Bedeutungen haben, umsetzt, gegebenenfalls Estergruppen hydrolytisch oder hydrogenolytisch abspaltet oder gegebenenfalls freie Carboxygrupen in an sich bekannter Weise verestert. 2. Verfahren gemäß Anspruch 1, dadurch gekennzeichnet,

daß
n = 1 oder 2 ist
R Wasserstoff,
Alkyl mit 2-8 C-Atomen,
Alkenyl mit 2-6 C-Atomen,
Cycloalkyl mit 3-9 C-Atomen,
Aryl mit 6-12 C-Atomen,
das durch ($C_1$-$C_4$)-Alkyl, ($C_1$-$C_4$)-Alkoxy, Hydroxy, Halogen, Nitro, Amino, Aminomethyl($C_1$-$C_4$)-Alkylamino, Di-($C_1$-$C_4$)-alkylamino, ($C_1$-$C_4$)-Acylamino, vorzugsweise ($C_1$-$C_4$)-Alkanoylamino, Methylendioxy, Carboxy, Cyano und/oder Sulfamoyl mono-, di- oder trisubstituiert sein kann,
Alkoxy mit 1-4 C-Atomen,
Aryloxy mit 6-12 C-Atomen,
das wie oben bei Aryl beschrieben substituiert sein kann,
mono bzw. bicyclisches Heteroaryloxy mit 5-7 bzw. 8-10 Ringatomen, wovon 1 bis 2 Ringatome Schwefeloder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)-alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxy-carbonylamino-($C_1$-$C_4$)-alkyl
($C_6$-$C_{12}$)-Aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,

$(C_6-C_{12})$-Arylthio-$(C_1-C_4)$-alkyl,
das im Arylteil wie oben bei Aryl beschrieben, substituiert sein kann,
$(C_6-C_{12})$-Aryl-$(C_1-C_4)$-alkylthio,
das im Arylteil wie oben bei Aryl beschrieben, substituiert sein kann
Carboxy-$(C_1-C_4)$-alkyl,
Carboxy, Carbamoyl,
Carbamoyl-$(C_1-C_4)$-alkyl,
$(C_1-C_4)$-Alkoxy-carbonyl-$(C_1-C_4)$-alkyl,
$(C_1-C_4)$Alkoxycarbonyl
$(C_6-C_{12})$-Aryloxy-$(C_1-C_4)$-alkyl,
das im Arylteil wie oben bei Aryl beschrieben substituiert sein kann oder
$(C_6-C_{12})$-Aryl-$(C_1-C_4)$-alkoxy,
das im Arylteil wie oben bei Aryl beschrieben substituiert sein kann,
$R^1$ Wasserstoff,
Alkyl mit 1-6 C-Atomen,
Alkenyl mit 2-6 C-Atomen,
Alkinyl mit 2-6 C-Atomen,
Cycloalkyl mit 3-9 C-Atomen,
Cycloalkenyl mit 5-9 C-Atomen,
$(C_3-C_9)$-Cycloalkyl-$(C_1-C_4)$-alkyl,
$(C_5-C_9)$-Cycloalkenyl-$(C_1-C_4)$-alkyl,
gegebenenfalls teilhydriertes Aryl mit 6-12 C-Atomen, das wie oben bei R beschrieben substituiert sein kann,$(C_6-C_{12})$-Aryl-$(C_1-C_4)$-alkyl oder $(C_7-C_{13})$-Aroyl-$(C_1$ oder $C_2)$alkyl
die beide wie das vorstehende Aryl substituiert sein können,
mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die Seitenkette einer gegebenenfalls geschützten natürlich vorkommenden α-Aminosäure $R^1$-$CH(NH_2)$-COOH bedeuten,
$R^2$ und $R^3$ gleich oder verschieden sind und Wasserstoff,
Alkyl mit 1-6 C-Atomen,
Alkenyl mit 2-6 C-Atomen,
Di-$(C_1-C_4)$-alkylamino-$(C_1-C_4)$-alkyl,
$(C_1-C_5)$-Alkanoyloxy-$(C_1-C_4)$-alkyl,
$(C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy$(C_1-C_4)$-alkyl
Aryl mit 6-12 C-Atomen,
$(C_6-C_{12})$ Aryl-$(C_1-C_4)$-alkyl,
$(C_3-C_9)$ Cycloalkyl oder
$(C_3-C_9)$-Cycloalkyl-$(C_1-C_4)$-alkyl
bedeuten.

3. Verfahren gemäß Anspruch 1, dadurch gekennzeichnet, daß
n = 1 oder 2 ist,
R $(C_2$ bis $C_6)$-Alkyl $(C_2$ bis $C_6)$-Alkenyl,
$(C_3$ bis $C_9)$-Cycloalkyl, Amino-$(C_1-C_4)$-alkyl,
$(C_2-C_5)$-Acylamino-$(C_1-C_4)$-alkyl, $(C_1-C_4)$
Alkoxycarbonylamino-$(C_1-C_4)$alkyl, $(C_6-C_{12})$
Aryl-$(C_1-C_4)$-alkoxycarbonylamino-$(C_1-C_4)$-alkyl,
$(C_7-C_{13})$-Aroylamino-$(C_1-C_4)$-alkyl, $(C_6$ bis $C_{12})$-Aryl, das durch $(C_1$ bis $C_4)$-Alkyl, $(C_1$ bis $C_4)$-Alkoxy, Hydroxy, Halogen, Nitro, Amino,
$(C_1$ bis $C_4)$-Alkylamino, Di-$(C_1$ bis $C_4)$alkylamino
und/oder Methylendioxy mono-, di- oder trisubstituiert sein kann, oder 3-Indolyl,
$R^1$ Wasserstoff oder $(C_1$ bis $C_6)$-Alkyl, das gegebenenfalls durch Amino, $(C_1$ bis $C_6)$-Acylamino oder Benzoylamino substituiert sein kann, $(C_2$ bis $C_6)$-Alkenyl, $(C_3$ bis $C_9)$-Cycloalkyl, $(C_5$ bis $C_9)$-Cycloalkenyl, $(C_3$ bis $C_7)$-Cycloalkyl-$(C_1$ bis $C_4)$-alkyl, $(C_6$ bis $C_{12})$-Aryl oder teilhydriertes Aryl, das jeweils durch $(C_1$ bis $C_4)$-Alkyl, $(C_1$ oder $C_2)$-Alkoxy oder Halogen substituiert sein kann, $(C_6-C_{12})$-Aryl-$(C_1$ bis $C_4)$-alkyl oder $(C_7-C_{13})$-Aroyl-$(C_1$-$C_2)$alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure.

$R^2$ und $R^3$ gleiche oder verschiedene Reste Wasserstoff, $(C_1$ bis $C_6)$-Alkyl, $(C_2$ bis $C_6)$-Alkenyl oder $(C_6$ - $C_{12})$-Aryl-$(C_1$ bis $C_4)$-alkyl,
bedeuten.

4. Verfahren gemäß Anspruch 1, dadurch gekennzeichnet, daß
n = 1 oder 2 ist, R Ethyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, Cyclohexyl oder Phenyl, das durch Phenyl, ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkyl-amino, Nitro und/ oder Methylendioxy mono- oder disubstituiert oder im Falle von Methoxy, trisubstituiert sein kann,
$R^1$ Wasserstoff, ($C_1$ bis $C_3$)-Alkyl, ($C_2$ oder $C_3$)-Alkenyl, die gegebenenfalls geschützte Seitenkette von Lysin, Benzyl, 4-Methoxybenzyl, 4-Ethoxy-benzyl, Phenethyl, 4-Amino-butyl oder Benzoylmethyl und
$R^2$ und $R^3$ gleiche oder verschiedene Reste Wasserstoff ($C_1$ bis $C_4$)-Alkyl oder Benzyl
bedeuten.

5. Verfahren zur Herstellung von verbindungen der Formel II

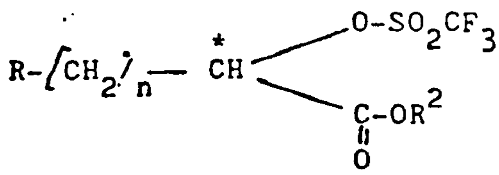

(II)

in welcher
n = 2 ist,
R ($C_1$ bis $C_6$)-Alkyl ($C_2$ bis $C_6$)-Alkenyl ($C_3$ bis $C_9$)-Cycloalkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino-$(C_1-C_4)$-alkyl, $(C_6-C_{12})$-Aryl-$(C_1-C_4)$-alkoxycarbonylamino-$(C_1-C_4)$-alkyl, ($C_6$ bis $C_{12}$)-Aryl, das durch ($C_1$ bis $C_4$)-Alkyl, ($C_1$ bis $C_4$)-Alkoxy-Hydroxy, Halogen, Nitro, Amino, ($C_1$ bis $C_4$)-Alkylamino, Di-($C_1$ bis $C_4$)alkyl-amino und/oder Methylendioxy mono-, di- oder trisubstituiert sein kann, oder 3-Indolyl und
$R^2$ Wasserstoff, ($C_1$bis $C_6$)-Alkyl, ($C_2$ bis $C_6$)-Alkenyl oder ($C_6$ bis $C_{12}$)-Aryl- ($C_1$ bis $C_4$)-alkyl
bedeuten,
dadurch gekennzeichnet, daß man ein α-Hydroxycarbonsäurederivat der Formel VI

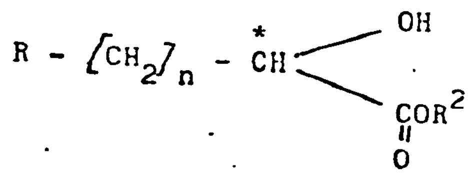

(VI)

in welcher n, R und $R^2$ die oben genannten Bedeutungen haben, mit einem Trifluormethansulfonierungsmittel gegebenenfalls in Gegenwart einer Base umsetzt.

6. Verfahren zur Herstellung von Verbindungen der Formel III

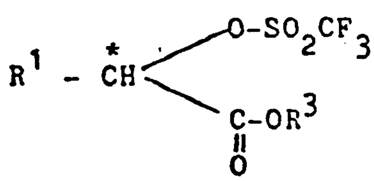

(III)

in welcher
$R^1$ Wasserstoff oder ($C_2$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_3$ bis $C_9$)-Cycloalkyl, ($C_5$ bis $C_9$)-Cycloalkenyl, ($C_3$ bis $C_7$)-Cycloalkyl-($C_1$ bis $C_4$)-alkyl, ($C_6$ bis $C_{12}$)-Aryl oder teilhydriertes Aryl, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_2$ bis $C_4$)-alkyl oder ($C_7$ - $C_{13}$)-Aroyl-($C_1$-$C_2$)alkyl die beide wie vorstehend definiert im Arylrest substituiert sein können, ein mono- bzw. bicyclischer

Heterocyclen-Rest mit 5 bis 7 bzw 8 bis 10 Ringatomen, wovon 1 bis 2 Ringatome Schwefel- oder Bauerstoffatome und/oder 1 bis 4 Ringatome Stickstoffatome darstellen, oder eine Seitenkette einer natürlich vorkommenden, geschützten α-Aminosäure und

$R^3$ Wasserstoff, ($C_1$ bis $C_6$)-Alkyl, ($C_2$ bis $C_6$)-Alkenyl oder ($C_6$ - $C_{12}$)-Aryl-($C_1$ bis $C_4$)-alkyl bedeuten, dadurch gekennzeichnet, daß man ein α -Hydroxycarbonsäurederivat der Formel VII

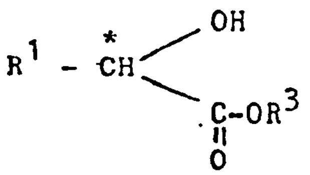

(VII)

in welcher $R^1$ und $R^3$ die oben genannten Bedeutungen haben, mit einem Trifluormethansulfonierungsmittel gegebenenfalls in Gegenwart einer Base umsetzt.

7. Verfahren gemäß Anspruch 5, dadurch gekennzeichnet, daß

R Ethyl, tert.Butoxycarbonylamino-($C_1$-$C_4$)-alkyl, Benzyloxycarbonylamino-($C_1$-$C_4$)-alkyl, Cyclohexyl oder Phenyl, das durch Phenyl, ($C_1$ oder $C_2$)-Alkyl, ($C_1$ oder $C_2$)-Aikoxy, Hydroxy, Fluor, Chlor, Brom, Amino, ($C_1$ bis $C_4$)-Alkylamino, Di-($C_1$ bis $C_4$)-alkylamimo, Nitro und/oder Methylendioxy mono- oder disubstituiert oder im Falle von Methoxy, trisubstituiert sein kann, und

$R^2$ Wasserstoff, ($C_1$ bis $C_4$)-Alkyl oder Benzyl bedeuten.

8. Verfahren gemäß Anspruch 6, dadurch gekennzeichnet, daß

$R^1$ Wasserstoff, ($C_2$ oder $C_3$)-Alkyl, ($C_2$ oder $C_3$)-Alkenyl, die geschützte Seitenkette von Lysin, 4-Methoxybenzyl, 4-Ethoxybenzyl, Phenethyl, 4-Amino-butyl oder Benzoylmethyl und

$R^3$ Wasserstoff, ($C_1$ bis $C_4$)-Alkyl oder Benzyl bedeuten.

9. Verfahren gemäß Anspruch 5, dadurch gekennzeichnet, daß

R Phenyl oder mit Fluor und/oder Chlor mono- oder disubstituiertes Phenyl und

$R^2$ Wasserstoff, Methyl, Ethyl, tert.Butyl oder Benzyl

bedeuten.

10. verfahren gemäß Anspruch 6, dadurch gekennzeichnet, daß

$R^1$ die acylierte Seitenkette von Lysin oder die O-($C_1$ - $C_6$) – alkylierte Seitenkette von Tyrosin und

$R^3$ Wasserstoff, Methyl, Ethyl, tert. Butyl oder Benzyl

bedeuten.

**Claims** for the Contracting states: BE, CH, DE, FR, GB, IT, LI, LU, NL, SE

1. A process for preparing compounds of the formula I

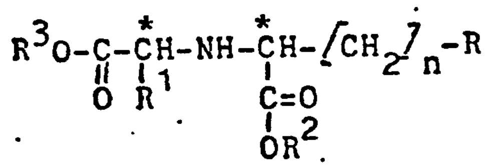

(I)

in which

n = 1 or 2,

R denotes hydrogen,

carboxy, carbamoyl,

an optionally substituted aliphatic radical having 2-8 carbon atoms,

an optionally substituted cycloaliphatic radical having 3-9 carbon atoms, an optionally substituted aromatic radical having 6-12 carbon atoms,

an $OR^4$ or $SR^4$ radical in which

$R^4$ represents an optionally substituted aliphatic radical having 1-4 carbon atoms, an optionally substituted aromatic radical having 6-12 carbon atoms or an optionally substituted heteroaromatic radical having 5-10 ring atoms, or

an optionally substituted heteroaromatic radical having 5-10 ring atoms,

$R^1$ denotes hydrogen,

an optionally substituted aliphatic radical having 1-6 carbon atoms,
an optionally substituted cycloaliphatic radical having 3-9 carbon atoms,
an optionally substituted cycloaliphatic-aliphatic radical having 4-13 carbon atoms, an optionally substituted aromatic radical having 6-12 carbon atoms,
an optionally substituted araliphatic radical having 7-16 carbon atoms,
an optionally substituted heteroaromatic radical having 5-10 ring atoms or
the side chain of an optionally protected naturally occurring α-amino acid,
$R^2$ and $R^3$ are identical or different and denote hydrogen,
an optionally substituted aliphatic radical having 1-6 carbon atoms,
an optionally substituted cycloaliphatic radical having 3-9 carbon atoms,
an optionally substituted cycloaliphatic-aliphatic radical having 4-12 carbon atoms,
an optionally substituted aromatic radical having 6-12 carbon atoms or
an optionally substituted araliphatic radical having 7-16 carbon atoms,
which comprises reacting compounds of the formulae II or III

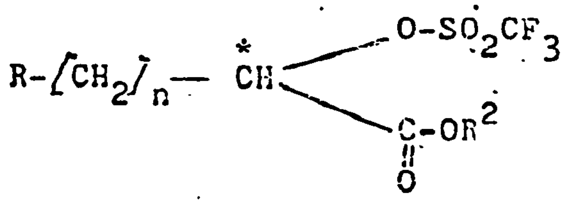

(II)

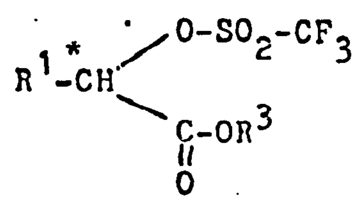

(III)

in which n, R, $R^1$, $R^2$ and $R^3$ have the abovementioned meanings, with compounds of the formulae IV or V

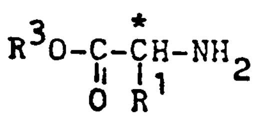

(IV)

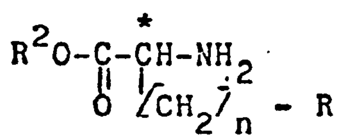

(V)

in which R, $R^1$, $R^2$, $R^3$ and n have the abovementioned meanings, if desired eliminating ester groups by hydrolysis or hydrogenolysis or if desired esterifying free carboxyl groups in a manner known per se.

2. The process as claimed in claim i, wherein
n 1 or 2,
R denotes hydrogen,
alkyl having 2-8 carbon atoms,
alkenyl having 2-6 carbon atoms,
cycloalkyl having 3-9 carbon atoms,
aryl which has 6-12 carbon atoms
and can be monosubstituted, disubstituted or trisubstituted by $(C_1$-$C_4)$-alkyl, $(C_1$-$C_4)$-alkoxy, hydroxyl, halogen, nitro, amino, aminomethyl, $(C_1$-$C_4)$-alkylamino, di-$(C_1$-$C_4)$-alkylamino, $(C_1$-$C_4)$-acylamino, preferably $(C_1$-$C_4)$-alkanoyl-amino, methylenedioxy, carboxyl, cyano and/or sulfamoyl,
alkoxy having 1-4 carbon atoms,
aryloxy which has 6-12 carbon atoms
and which can be substituted as described above for aryl,
monocyclic or bicyclic heteroaryloxy which has 5-7 or 8-10 respectively ring atoms of which 1 or 2 ring atoms represent sulfur or oxygen atoms and/or
1 to 4 ring atoms represent nitrogen
and which can be substituted as described above for aryl,
amino-$(C_1$-$C_4)$-alkyl,
$(C_1$-$C_4)$-alkanoylamino-$(C_1$-$C_4)$-alkyl,
$(C_7$-$C_{13})$-aroylamino-$(C_1$-$C_4)$-alkyl,
$(C_1$-$C_4)$-alkoxycarbonylamino-$(C_1$-$C_4)$-alkyl,
$(C_6$-$C_{12})$-aryl-$(C_1$-$C_4)$-alkoxycarbonylamino-$(C_1$-$C_4)$-alkyl,
$(C_6$-$C_{12})$-aryl-$(C_1$-$C_4)$-alkylamino-$(C_1$-$C_4)$-alkyl,
$(C_1$-$C_4)$-alkylamino-$(C_1$-$C_4)$-alkyl,

di-($C_1$-$C_4$)-alkylamino-($C_1$-$C_4$)-alkyl,
guanidino-($C_1$-$C_4$)-alkyl,
imidazolyl, indolyl,
($C_1$-$C_4$)-alkylthio,
($C_1$-$C_4$)-alkylthio-($C_1$-$C_4$)-alkyl,
($C_6$-$C_{12}$)-arylthio-($C_1$-$C_4$)-alkyl
which can be substituted in the aryl moiety as described above for aryl,
($C_6$-$C_{12}$)-aryl-($C_1$-$C_4$)-alkylthio
which can be substituted in the aryl moiety as described above for aryl,
carboxy-($C_1$-$C_4$)-alkyl,
carboxyl, carbamoyl,
carbamoyl-($C_1$-$C_4$)-alkyl,
($C_1$-$C_4$)-alkoxycarbonyl-($C_1$-$C_4$)-alkyl,
($C_1$-C4)-alkoxycarbonyl,
($C_6$-$C_{12}$)-aryloxy-($C_1$-$C_4$)-alkyl
which can be substituted in the aryl moiety as described above for aryl or
($C_6$-$C_{12}$)-aryl-($C_1$-$C_4$)-alkoxy
which can be substituted in the aryl moiety as described above for aryl,
$R^1$ denotes hydrogen,
alkyl having 1-6 carbon atoms,
alkenyl having 2-6 carbon atoms,
alkynyl having 2-6 carbon atoms,
cycloalkyl having 3-9 carbon atoms,
cycloalkenyl having 5-9 carbon atoms,
($C_3$-$C_9$)-cycloalkyl-($C_1$-$C_4$)-alkyl,
($C_5$-$C_9$)-cycloalkenyl-($C_1$-$C_4$)-alkyl,
optionally partially hydrogenated aryl which has 6-12 carbon atoms and can be substituted as described above for R, ($C_6$-$C_{12}$)-aryl-($C_1$-$C_4$)-alkyl or ($C_7$-$C_{13}$)-aroyl-($C_1$ or $C_2$)-alkyl
either of which can be substituted like the aryl above,
monocyclic or bicyclic optionally partially hydrogenated heteroaryl which has 5-7 or 8-10 ring atoms respectively, of which 1 or 2 ring atoms represent sulfur or oxygen atoms and/or 1 to 4 ring atoms represent nitrogen atoms,
and which can be substituted like aryl above, or the side chain of an optionally protected naturally occurring α-amino acid of the formula $R^1$-CH($NH_2$)-COOH,
$R^2$ and $R^3$ are identical or different and denote hydrogen,
alkyl having 1-6 carbon atoms,
alkenyl having 2-6 carbon atoms,
di-($C_1$-$C_4$)-alkylamino-($C_1$-$C_4$)-alkyl,
($C_1$-$C_5$)-alkanoyloxy-($C_1$ – C4)-alkyl,
($C_1$-$C_6$) – alkoxycarbonyloxy – ($C_1$-$C_4$) – alkyl,
($C_7$-$C_{13}$)-aroyloxy-($C_1$-$C_4$)-alkyl,
($C_6$-$C_{12}$)-aryloxycarbonyloxy-($C_1$-$C_4$)-alkyl,
aryl having 6-12 carbon atoms,
($C_6$-$C_{12}$)-aryl-($C_1$-$C_4$)-alkyl,
($C_3$-$C_9$)-cycloalkyl or
($C_3$-$C_9$-cycloalkyl-($C_1$-$C_4$)-alkyl.

3. The process as claimed in claim 1, wherein
n = 1 or 2,
R denotes ($C_2$ to $C_6$)-alkyl, ($C_2$ to $C_6$)-alkenyl,
($C_3$ to $C_9$)-cycloalkyl, amino-($C_1$-$C_4$)-alkyl, ($C_2$-$C_5$)-acylamino-($C_1$-$C_4$)-alkyl, ($C_1$-$C_4$)-alkoxy-carbonylamino-($C_1$-$C_4$)-alkyl, ($C_6$ to $C_{12}$)-aryl-($C_1$-$C_4$)-alkoxy-carbonylamino-($C_1$-$C_4$)-alkyl, ($C_7$-$C_{13}$)-aroylamino-($C_1$-$C_4$)-alkyl, ($C_6$ to $C_{12}$)-aryl
which can be monosubstituted, disubstituted or trisubstituted by ($C_1$ to $C_4$)-alkyl, ($C_1$ to $C_4$)-alkoxy, hydroxyl, halogen, nitro, amino, ($C_1$ to $C_4$)-alkylamino, di-($C_1$-$C_4$)-alkylamino or methyl-enedioxy, and/or 3-indolyl,
$R^1$ denotes hydrogen or ($C_1$ to $C_6$)-alkyl which can be optionally substituted by amino ($C_1$ $C_6$)-acylamino or benzoylamino, ($C_2$ to $C_6$)-alkenyl, ($C_3$ to $C_9$)-cycloalkyl, ($C_5$ to $C_9$)-cycloalkenyl, ($C_3$ to $C_7$)-cycloalkyl-($C_1$ to $C_4$)-alkyl, ($C_6$ to $C_{12}$)-aryl or partially hydrogenated aryl, either of which can be substituted by ($C_1$ to $C_4$)-alkyl, ($C_1$ or $C_2$)-alkoxy or halogen, ($C_6$-$C_{12}$) – aryl-($C_1$ to $C_4$)-alkyl or,($C_7$-$C_{13}$)-aroyl-($C_1$-$C_2$)-alkyl, both of which can be substituted in the aryl radical as defined above, a monocyclic or bicyclic heterocyclic radical having 5 to 7 or 8 to 10 ring atoms respectively, of which 1 or 2 ring atoms represent sulfur or oxygen atoms and/or 1 to 4 ring atoms represent nitrogen atoms, or a side chain of a naturally occurring optionally protected α-amino acid,
$R^2$ and $R^3$ denote identical or different radicals from among hydrogen ($C_1$ to $C_6$)-alkyl, ($C_2$ to $C_6$)-alkenyl and ($C_6$ to $C_{12}$)-aryl-($C_1$ to $C_4$)-alkyl.

4. The process as claimed in claim 1, wherein
n = 1 or 2,

R denotes ethyl, $(C_1-C_4)$-alkoxycarbonylamino-$(C_1-C_4)$-alkyl, $(C_6-C_{12})$-aryl-$(C_1-C_4)$-alkoxycarbonyl-amino-$(C_1-C_4)$-alkyl, cyclohexyl or phenyl, which can be monosubstituted or disubstituted by phenyl, ($C_1$ or $C_2$)-alkyl, ($C_1$ or $C_2$)-alkoxy, hydroxyl, fluorine, chlorine, bromine, amino, ($C_1$ to $C_4$)-alkylamino, di-($C_1$ to $C_4$)-alkylamino, nitro and/ or methylenedioxy or, in the case of methoxy, trisubstituted,

$R^1$ denotes hydrogen, ($C_1$ to $C_3$)-alkyl, ($C_2$ or $C_3$)-alkenyl, the optionally protected side chain of lysine, benzyl, 4-methoxybenzyl, 4-ethoxybenzyl, phenethyl, 4-aminobutyl or benzoylmethyl, and

$R^2$ and $R^3$ denote identical or different radicals from among hydrogen, ($C_1$ to $C_4$)-alkyl and benzyl.

5. A compound of the formula II

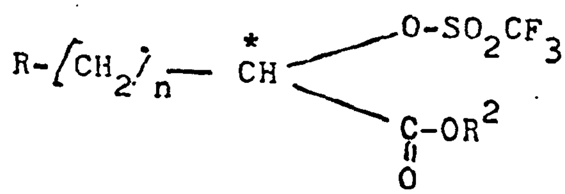

(II)

in which

n = 2,

R denotes ($C_1$ to $C_6$)-alkyl, ($C_2$ to $C_6$)-alkenyl, ($C_3$ to $C_9$)-cycloalkyl, $(C_2-C_5)$-acylamino-$(C_1-C_4)$-alkyl, $(C_7-C_{13})$-aroylamino-$(C_1-C_4)$ – alkyl, $(C_1-C_4)$-alkoxycarbonylamino-$(C_1-C_4)$-alkyl, $(C_6-C_{12})$-aryl-$(C_1-C_4)$-alkoxycarbonylamino-$(C_1-C_4)$-alkyl, ($C_6$ to $C_{12}$)-aryl which can be monosubstituted, disubstituted or trisubstituted by ($C_1$ to $C_4$)-alkyl, ($C_1$ to $C_4$)-alkoxy hydroxy halogen, nitro, amino, ($C_1$ to $C_4$)-alkylamino, di-($C_1$ to $C_4$)-alkylamino and/ or methylenedioxy, or 3-indolyl, and

$R^2$ denotes hydrogen, ($C_1$ to $C_6$)-alkyl, ($C_2$ to $C_6$)-alkenyl or ($C_6$ to $C_{12}$)-aryl, ($C_1$ to $C_4$)-alkyl.

6. A compound of the formula III

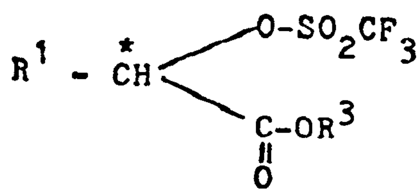

(III)

in which

$R^1$ denotes hydrogen or ($C_2$ to $C_6$)-alkyl, which can be optionally substituted by amino, ($C_1$ to $C_6$)-acyl-amino or benzoylamino, ($C_2$ to $C_6$)-alkenyl, ($C_3$ to $C_9$)-cycloalkyl, ($C_5$ to $C_9$)-cycloalkenyl, ($C_3$ to $C_7$)-cycloalkyl-($C_1$ to $C_4$)-alkyl, ($C_6$ to $C_{12}$)-aryl or partially hydrogenated aryl, either of which can be substituted by ($C_1$ to $C_4$)-alkyl, ($C_1$ or $C_2$)-alkoxy or halogen, ($C_6$ to $C_{12}$)-aryl-($C_2$ to $C_4$)-alkyl or $(C_7-C_{13})$-aroyl-$(C_1-C_2)$-alkyl, either of which can be substituted in the aryl radical as defined above, a monocyclic or bicyclic heterocyclic radical having 5 to 7 or 8 to 10 ring atoms respectively of which 1 or 2 ring atoms represent sulfur or oxygen atoms and/or 1 to 4 ring atoms represent nitrogen atoms, or a side chain of a naturally occurring protected αamino acid, and

$R^3$ denotes hydrogen, ($C_1$ to $C_6$)-alkyl, ($C_2$ to $C_6$)-alkenyl or $(C_6-C_{12})$-aryl-($C_1$ to $C_4$)-alkyl.

7. A compound as claimed in claim 5, wherein

R denotes ethyl, tert.-butoxycarbonylamino-$(C_1-C_4)$-alkyl, benzyloxycarbonylamino-$(C_1-C_4)$-alkyl, cyclohexyl or phenyl which can be monosubstituted or disubstituted by phenyl, ($C_1$ or $C_2$)-alkyl, ($C_1$ or $C_2$)-alkoxy, hydroxyl, fluorine, chlorine, bromine, amino, ($C_1$ to $C_4$)-alkylamino, di-($C_1$ to $C_4$)-alkylamino, nitro and/or methylenedioxy or, in the case of methoxy, trisubstituted, and

$R^2$ denotes hydrogen, ($C_1$ to $C_4$)-alkyl or benzyl.

8. A compound as claimed in claim 6, wherein

$R^1$ denotes hydrogen, ($C_2$ or $C_3$)-alkyl, ($C_2$ or $C_3$)-alkenyl, the protected side chain of lysine, 4-methoxybenzyl, 4-ethoxybenzyl, phenethyl, 4-aminobutyl or benzoylmethyl and

$R^3$ denotes hydrogen, ($C_1$ to $C_4$)-alkyl or benzyl.

9. A compound as claimed in claim 5, wherein

R denotes phenyl or fluorine- and/or chlorine-monosubstituted or -disubstituted phenyl and
$R^2$ denotes hydrogen, methyl, ethyl, tert.-butyl or benzyl.

10. A compound as claimed in claim 6, wherein
$R^1$ denotes the acylated side chain of lysine or the O-($C_1$-$C_6$)-alkylated side chain of tyrosine and
$R^3$ denotes hydrogen, methyl, ethyl, tert.-butyl or benzyl.

11. A process for preparing compounds of the formulae II or III, which comprises reacting α-hydroxycarboxylic acid derivatives of the formulae VI or VII

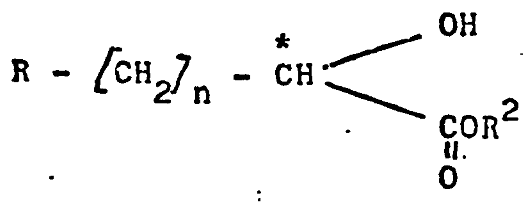

(VI)

(VII)

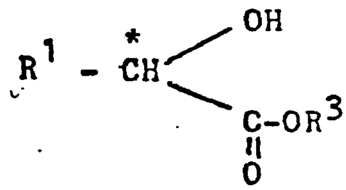

in which n, R, $R^1$, $R^2$ and $R^3$ have the abovementioned meanings, with a trifluoromethanesulfonating agent in the presence or absence of a base.

**Claims** for the Contracting state: Austria

1. A process for preparing compounds of the formula

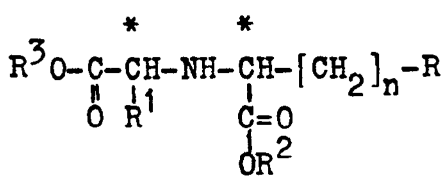

(I)

in which
n = 1 or 2,
R denotes hyqrogen, carboxy, carbamoyl, an optionally substituted aliphatic radical having 2 - 8 carbon atoms,
an optionally substituted cycloaliphatic radical having 3 - 9 carbon atoms, an optionally substituted aromatic radical having 6 - 12 carbon atoms, an $OR^4$ or $SR^4$ radical in which
$R^4$ reprrsents an optionally substituted aliphatic radical having 1 - 4 carbon atoms, an optionally substituted aromatic radical having 6 - 12 carbon atoms or an optionally substituted heteroaromatic radical having 5 - 10 ring atoms, or
an optionally substituted heteroaromatic radical having 5 - 10 ring atoms,
$R^1$ denotes hydrogen,
an optionally substituted aliphatic radical having 1 - 6 carbon atoms,
an optionally substituted cycloaliphatic radical having 3 - 9 carbon atoms,
an optionally substituted cycloaliphatic-aliphatic radical having 4 - 13 carbon atoms,
an optionally substituted aromatic radical having 6 - 12 carbon atoms,
an optionally substituted araliphatic radical having 7 - 16 carbon atoms,
an optionally substituted heteroaroamtic radical having 5 - 10 ring atoms or
the side chain of an optionally protected naturally occuring α-amino acid,

$R^2$ and $R^3$ are identical or different and denote hydrogen, an optionally substituted aliphatic radical having 1 - 6 carbon atoms,
an optionally substituted cycloaliphatic radical having 3 - 9 carbon atoms,
an optionally substituted cycloaliphatic-aliphatic radical having 4 - 12 carbon atoms,
an optionally substituted aromatic radical having 6 - 12 carbon atoms or
an optionally substituted araliphatic radical having 7 - 16 carbon atoms,
which comprises reacting compounds of the formulae II or III

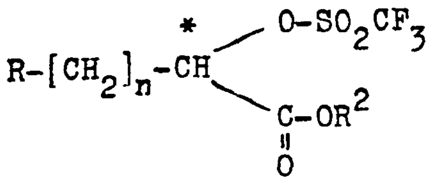

(II)

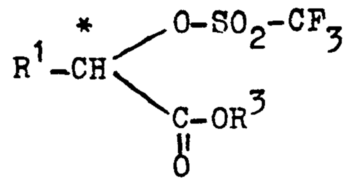

(III)

in which n, R, $R^1$, $R^2$ and $R^3$ have the abovementioned meanings, with compounds of the formulae IV or V

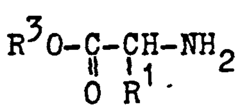

(IV)

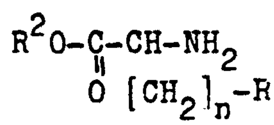

(V)

in which R, $R^1$, $R^2$, $R^3$ and n have the abovementioned meanings, if desired eliminating ester groups by hydrolysis or hydrogenolysis or if desired esterifying free carboxyl groups in a manner known per se.

2. The process as claimed in claim 1, wherein
n = 1 or 2,
R denotes hydrogen,
alkyl having 2 - 8 carbon atoms,
alkenyl having 2 - 6 carbon atoms,
cycloalkyl having 3 - 9 carbon atoms,
aryl which has 6 - 12 carbon atoms
and can be monosubstituted, disubstituted or trisubstituted by $(C_1-C_4)$-alkyl, $(C_1-C_4)$-alkoxy, hydroxyl, halogen, nitro, amino, aminomethyl, $(C_1-C_4)$-alkylamino, di-$(C_1-C_4)$-alkylamino, $(C_1-C_4)$-acylamino, prrferably $(C_1-C_4)$-alkanoylamino, methylenedioxy, carboxyl, cyano and/or sulfamoyl,
alkoxy having 1 - 4 carbon atoms,
aryloxy which has 6 - 1 2 carbon atoms
and which can be substituted as described above for aryl,
monocyclic or bicyclic heteroaryloxy which has 5 - 7 or 8 - 10 respectively ring atoms of which 1 or 2 ring atoms represent sulfur or oxygen atoms and/or 1 to 4 ring atoms rrpresent nitrogen
and which can be substituted as described above for aryl,
amino-$(C_1-C_4)$-alkyl,
$(C_1-C_4)$-alkanoylamino-$(C_1-C_4)$-alkyl,
$(C_7-C_{13})$-aroylamino-$(C_1-C_4)$-alkyl,
$(C_1-C_4)$-alXoxycarbonylamin o-$(C_1-C_4)$-alkyl,
$(C_6-C_{12})$-aryl-$(C_1-C_4)$-alkoxycarbonylamino-$(C_1-C_4)$-alkyl,
$(C_6-C_{12})$-aryl-$(C_1-C_4)$-alkylamino-$(C_1-C_4)$-alkyl,
$(C_1-C_4)$-alkylamino-$(C_1-C_4)$-alkyl,
di-$(C_1-C_4)$-alkylamino-$(C_1-C_4)$-alkyl,
guanidino-$(C_1-C_4)$-alkyl,
imidazolyl, indolyl,
$(C_1-C_4)$-alkylthio,
$(C_1-C_4)$-alkylthio-$(C_1-C_4)$-alkyl,
$(C_6-C_{12})$-arylthio-$(C_1-C_4)$-alkyl
which can be substitued in the aryl moiety as described above for aryl,
$(C_6-C_{12})$-aryl-$(C_1-C_4)$-alkylthio

which can be substituted in the aryl moiety as described above for aryl,
carboxy-($C_1$-$C_4$)-alkyl,
carboxy, carbamoyl,
carbamoyl-($C_1$-$C_4$)-alkyl,
($C_1$-$C_4$)-alkoxycarbonyl-($C_1$-$C_4$)-alkyl,
($C_1$-$C_4$)-alkoxycarbonyl,
($C_6$-$C_{12}$)-aryloxy-($C_1$-$C_4$)-alkyl
which can be substituted in the aryl moiety as described above for aryl or
($C_6$-$C_{12}$)-aryl-($C_1$-$C_4$)-alkoxy
which can be substituted in the aryl moiety as described above for aryl,
$R^1$ denotes hydrogen,
alkyl having 1 - 6 carbon atoms,
alkenyl having 2 - 6 carbon atoms,
alkynyl having 2 - 6 carbon atoms,
cycloalkyl having 3 - 9 carbon atoms,
cycloalkenyl having 5 - 9 carbon atoms,
($C_3$-$C_9$)-cycloalkyl-($C_1$-$C_4$)-alkyl,
($C_5$-$C_9$)-cycloalkenyl-($C_1$-$C_4$)-alkyl,
optionally partially hydrogenated aryl which has 6 - 12 carbon atoms and can be substituted as described above for R, ($C_6$-$C_{12}$)-aryl-($C_1$-$C_4$)-alkyl or ($C_7$-$C_{13}$)-aroyl-($C_1$ or $C_2$)-alkyl
either of which can be substituted like the aryl above,
monocyclic or bicyclic optionally partially hydrogenated heteroaryl which has 5 - 7 or 8 - 10 ring atoms respectively, of which 1 or 2 ring atoms represent sulfur or oxygen atoms and/or 1 to 4 ring atoms represent nitrogen atoms,
and which can be substituted like aryl above, or the side chain of an optionally protected naturally occurring α-amino acid of the formula $R^1$-CH($NH_2$) COOH,
$R^2$ and $R^3$ are identical or different and denote hydrogen,
alkyl having 1 - 6 carbon atoms,
alkenyl having 2 - 6 carbon atoms,
di-($C_1$-$C_4$)-alkylamino-($C_1$-$C_4$)-alkyl,
($C_1$-$C_5$)-alkan oyloxy-($C_1$-$C_4$)-alkyl,
($C_1$-$C_6$)-alkoxycarbonyloxy-($C_1$-$C_4$)-alkyl,
($C_7$-$C_{13}$)-aroyloxy-($C_1$-$C_4$)-alkyl,
($C_6$-$C_{12}$)-aryloxycarbonyloxy-($C_1$-$C_4$)-alkyl,
aryl having 6 - 12 carbon atoms,
($C_6$-$C_{12}$)-aryl-($C_1$-$C_4$)-alkyl,
($C_3$-$C_9$)-cycloalkyl or
($C_3$-$C_9$)-cycloalkyl-($C_1$-$C_4$)-alkyl.

3. The process as claimed in claim 1, wherein
n = 1 or 2,
R denotes ($C_2$ to $C_6$)-alkyl, ($C_2$ to $C_6$)-alkenyl, ($C_3$ to $C_9$)-cycloalkyl, amino-($C_1$-$C_4$)-alkyl, ($C_2$-$C_5$)-acylamin o-($C_1$-$C_4$)-alkyl, ($C_1$-$C_4$)-alkoxycarbonylamin o-($C_1$-$C_4$)-alkyl, ($C_6$ to $C_{12}$)-aryl-($C_1$-$C_4$)-alkoxy-carbonylamino-($C_1$-$C_4$)-alkyl, ($C_7$-$C_{13}$)-aroylamino-($C_1$-$C_4$)-alkyl, ($C_6$ to $C_{12}$)-aryl
which can be monosubstituted, disubstituted or trisubstituted by ($C_1$ to $C_4$)-alkyl, ($C_1$ to $C_4$)-alkoxy, hydroxyl, halogen, nitro, amino, ($C_1$ to $C_4$)-alkyl– amino, di-($C_1$-$C_4$)-alkylamino or methylenedioxy, and/or 3-indolyl,
$R^1$ denotes hydrogen or ($C_1$ to $C_6$)-alkyl which can beoptionally substituted by amino, ($C_1$ to $C_6$)-acylamino or benzoylamino, ($C_2$ to $C_6$)-alkenyl, ($C_3$ to $C_9$)-cycloalkyl, ($C_5$ to $C_9$)-cycloalkenyl, ($C_3$ to $C_7$)-cycloalkyl-($C_1$ to $C_4$)-alkyl, ($C_6$ to $C_{12}$)-aryl or parially hydrogenated aryl, either of which can be substituted by ($C_1$ to $C_4$) -alkyl, ($C_1$ or $C_2$)-alkoxy or halogen, ($C_6$-$C_{12}$)-aryl-($C_1$ to $C_4$)-alkyl or ($C_7$-$C_{13}$ aroyl-($C_1$-$C_2$)-alkyl, both of which can be substituted in the aryl radical as defined above, a monocyclic or bicyclic hetercyclic radical having 5 to 7 or 8 to 10 ring atoms respectively, of which 1 or 2 ring atoms represent sulfur or oxygen and/or 1 to 4 ring atoms represent nitrogen atoms, or a side chain of a naturally occurring optionally protected α-amino acid,
$R^2$ and $R^3$ denote identical or different radicals from among hydrogen, ($C_1$ to $C_6$)-alkyl, ($C_2$ to $C_6$)-alkenyl and ($C_6$ to $C_{12}$)-aryl-($C_1$ to $C_4$)-alkyl.

4. The process as claimed in claim 1, wherein
n = 1 or 2,
R denotes ethyl, ($C_1$-$C_4$)-alkoxycarbonylamino-($C_1$-$C_4$)-alkyl, ($C_6$-$C_{12}$)-aryl-($C_1$-$C_4$)-alkoxycarbonylamino-($C_1$-$C_4$)-alkyl, cyclohexyl or phenyl, which can be monosubstituted or disubstituted by phenyl, ($C_1$ or $C_2$)-alkyl, ($C_1$ or $C_2$)-alkoxy, hydroxyl, fluorine, chlorine, bromine, amino, ($C_1$ to $C_4$)-alkylamino, di-($C_1$ to $C_4$)-alkylamino, nitro and/or methylenedioxy or, in the case of methoxy, trisubstituted,
$R^1$ denotes hydrogen, ($C_1$ to $C_3$)-alkyl, ($C_2$ or $C_3$)-alkenyl, the optionally protected side chain of lysine, benzyl, 4-methoxybenzyl, 4-ethoxybenzyl, phenethyl, 4-aminobutyl or benzoylmethyl, and
$R^2$ and $R^3$ denote identical or different radicals from among hydrogen, ($C_1$ to $C_4$)-alkyl and benzyl.

5. A process for preparing compounds of the formula II

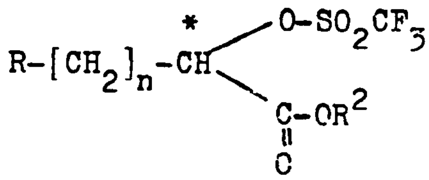

(II)

in which
n = 2,
R denotes ($C_1$ to $C_6$)-alkyl, ($C_2$ to $C_6$)-alkenyl, ($C_3$ to $C_9$)-cycloalkyl, ($C_2$-$C_5$)-acylamino-($C_1$ -$C_4$)-alkyl, ($C_7$-$C_{13}$)-aroylamino-($C_1$-$C_4$)-alkyl, ($C_1$-$C_4$)-alkoxycarbonylamino-($C_1$-$C_4$)-alkyl, ($C_6$ -$C_{12}$)-aryl-($C_1$-$C_4$)-alkoxycarbonylamino- ($C_1$-$C_4$)alkyl ($C_6$-$C_{12}$)-aryl which can be monosubstituted, disubstituted or trisubstituted by ($C_1$ to $C_4$)-alkyl, ($C_1$ to $C_4$)-alkoxy, hydroxy, halogen, nitro, amino, ($C_1$ to $C_4$)-alkylamino, di-($C_1$ to $C_4$)-aikylamino and/or methylenedioxy or 3-indolyl, and
$R^2$ denotes hydrogen, ($C_1$ to $C_6$)-alkyl, ($C_2$ to $C_6$)-alkenyl or ($C_6$ to $C_{12}$)-aryl, ($C_1$ to $C_4$)-alkyl, which comprises reacting α-hydroxycarboxylic acid derivatives of the formula VI

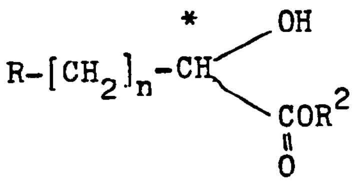

in which n, R and $R^2$ have the abovementioned meanings, with a trifluoromethanesulfonating agent in the presence or absence of a base.

6. A process for preparing compounds of the formula III

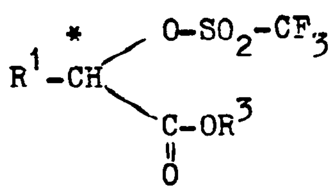

(III)

in which
$R^1$ denotes hydrogen or ($C_2$ to $C_6$)-alkyl, which can be optionally substituted by amino, ($C_1$ to $C_6$)-acylamino or benzoylamino, ($C_2$ to $C_6$)-alkenyl, ($C_3$ to $C_9$)-cycloalkyl, ($C_5$ to $C_9$)-cycloalkenyl, ($C_3$ to $C_7$)-cycloalkyl-($C_1$ to $C_4$)-alkyl, ($C_6$ to $C_{12}$)-aryl or partially hydrogenated aryl, either of which can be substituted by ($C_1$ to $C_4$)-alkyl, ($C_1$ or $C_2$)-alkoxy or halogen, ($C_6$ to $C_{12}$)-aryl-($C_2$ to $C_4$)-alkyl or ($C_7$-$C_{13}$)-aroyl-($C_1$-$C_2$)-alkyl, either of which can be substituted in the aryl radical as defined above, a monocyclic or bicyclic heterocyclic radical having 5 to 7 or 8 to 10 ring atoms respectively of which 1 or 2 ring atoms represent sulfur or oxygen atoms and/or 1 to 4 ring atoms represent nitrogen atoms, or a side chain of a naturally occurring protected αamino acid, and
$R^3$ denotes hydrogen, ($C_1$ to $C_6$)-alkyl, ($C_2$ to $C_6$)-alkenyl or ($C_6$-$C_{1\ 2}$)-aryl-($C_1$-$C_4$)-alkyl, which comprises reacting α-hydroxycarboxylic acid derivatives of the formula VII

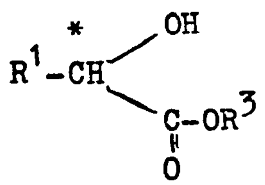

(VII)

in which $R^1$ and $R^3$ have the abovementioned meanings, with a trifluoromethanesulfonating agent in the presence or absence of a base.

7. A process as claimed in claim 5, wherein

R denotes ethyl, tert.-butoxycarbonylamino-($C_1$ -$C_4$)-alkyl, ben zyloxycarbonylamino-($C_1$ -$C_4$)-alkyl, cyclohexyl or phenyl which can be monosubstituted or disubstituted by phenyl, ($C_1$ or $C_2$)-alkyl, ($C_1$ or $C_2$)-alkoxy, hydroxyl, fluorine, chlorine, bromine, amino, ($C_1$-$C_4$)-alkylamino, di-($C_1$-$C_4$) alkylamino, nitro and/or methylenedioxy or, in the case of methoxy, trisubstituted, and

$R^2$ denotes hydrogen, ($C_1$ to $C_4$)-alkyl or benzyl.

8. A process as claimed in claim 6, wherein

$R^1$ denotes hydrogen, ($C_2$ or $C_3$)-alkyl, ($C_2$ or $C_3$)-alkenyl, the protected side chain of lysine, 4-methoxyben zyl, 4-ethoxyben zyl, phenethyl, 4-aminobutyl or benzoylmethyl and $R^3$ denotes hydrogen, ($C_1$ to $C_4$)-alkyl or benzyl.

9. A process as claimed in claim 5, wherein

R denotes phenyl or fluorine- and/or chlorinemonosubstituted or -disubstituted phenyl and

$R^2$ denotes hydrogen, methyl, ethyl, tert.-butyl or benzyl.

10. A process as claimed in claim 6, wherein

$R^1$ denotes the acylated side chain of lysine or the

O-($C_1$-$C_6$)-alkylated side chain of tyrosine and

$R^3$ denotes hydrogen, methyl, ethyl, tert.-butyl or benzyl.

**Revendications** pour les Etats contractants: BE, CH, DE, FR, GB, IT, LU, NL, SE

I. Procédé pour la préparation de composés de formule I

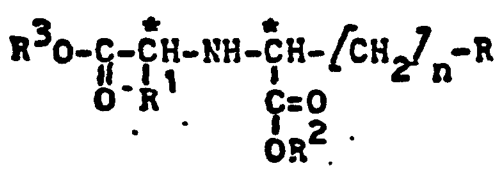

(I)

dans laquelle

n est 1 ou 2,

R représente l'hydrogène,

un groupe carboxy, carbamoyle,

un groupe aliphatique éventuellement substitué, ayant de 2 à 8 atomes de carbone,

un groupe cycloaliphatique éventuellement substitué ayant de 3 à 9 atomes de carbone, un groupe aromatique éventuellement substitué ayant de 6 à 12 atomes de carbone,

un groupe $OR^4$ ou $SR^4$ dans lequel

$R^4$ représente un groupe aliphatique éventuellement substitué ayant de 1 à 4 atomes de carbone,

un groupe aromatique éventuellement substitué ayant de 6 à 12 atomes de carbone ou un groupe hétéroaromatique éventuellement substitué ayant de 5 à 10 atomes dans le noyau, ou

un groupe hétéroaromatique éventuellement substitué ayant de 5 à 10 atomes dans le noyau,

$R^1$ représente l'hydrogène,

un groupe aliphatique éventuellement substitué, ayant de 1 à 6 atomes de carbone,

un groupe cycloaliphatique éventuellement substitué, ayant de 3 à 9 atomes de carbone,

un groupe cycloaliphatique-aliphatique, éventuellement substitué, ayant de 4 à 13 atomes de carbone,

un groupe aromatique éventuellement substitué, ayant de 6 à 12 atomes de carbone, un groupe araliphatique éventuellement substitué, ayant de 7 à 16 atomes de carbone, un groupe hétéroaromatique éventuellement substitué, ayant de 5 à 10 atomes dans le noyau, ou la chaîne latérale d'un acide α-aminé d'origine naturelle, éventuellement protégé,

$R^2$ et $R^3$ sont identiques ou différents et représentent l'hydrogène, un groupe aliphatique éventuellement substitué ayant de 1 à 6 atomes de carbone, un groupe cycloaliphatique éventuellement substitué ayant de 3 à 9 atomes de carbone, un groupe cycloaliphatique-aliphatique éventuellement substitué ayant de 4 à 12 atomes de carbone, un groupe aromatique éventuellement substitué ayant de 6 à 12 atomes de carbone, ou un groupe araliphatique éventuellement substitué ayant de 7 à 16 atomes de carbone,

ce procédé étant caractérisé en ce qu'on fait réagir des composés de formules II ou III

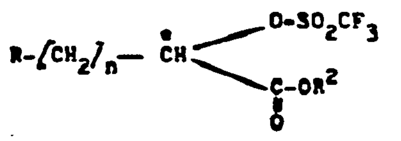

(II)

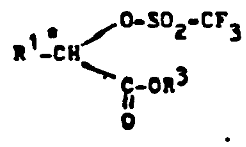

(III)

dans lesquelles n, R, $R^1$, $R^2$ et $R^3$ ont les significations indiquées ci-dessus, avec des composés de formules IV ou V

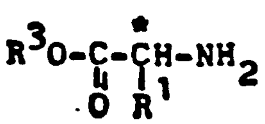

(IV)

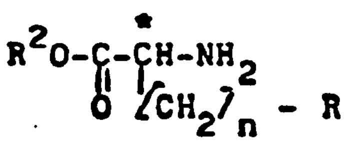

(V).

dans lesquelles R, $R^1$, $R^2$, $R^3$ et n ont les significations indiquées ci-dessus, on élimine éventuellement les groupes ester par hydrolyse ou hydrogènolyse ou bien on estérifie éventuellement les groupes carboxy libres, d'une manière connue en soi.

2. Procédé selon la revendication 1, caractérisé en ce que

n est 1 ou 2,

R représente l'hydrogène,

un groupe alkyle ayant de 2 à 8 atomes de carbone,

un groupe alcényle ayant de 2 à 6 atomes de carbone,

un groupe cycloalkyle ayant de 3 à 9 atomes de carbone,

un groupe aryle ayant de 6 à 12 atomes de carbone, qui peut être mono-, di- ou trisubstitué par un groupe alkyle ($C_1$-$C_4$), alcoxy ($C_1$-$C_4$), hydroxy, halogéno, nitro, amino, aminométhyl, alkyl ($C_1$-$C_4$)-amino, di-alkyl ($C_1$-$C_4$)-amino, acyl($C_1$-$C_4$)-amino, de préférence alcanoyl($C_1$-$C_4$)-amino, méthylènedioxy, carboxy, cyano et/ou sulfamoyle,

un groupe alcoxy ayant de 1 à 4 atomes de carbone,

un groupe aryloxy ayant de 6 à 12 atomes de carbone, qui peut être substitué comme décrit ci-dessus pour un groupe aryle,

un groupe hétéroaryloxy mono- ou bicyclique, ayant de 5 à 7 ou de 8 à 10 atomes de carbone dans le noyau, dont de 1 à 2 atomes du noyau représentent des atomes de soufre ou d'oxygène et/ou de 1 à 4 atomes du noyau représentent des atomes d'azote.

qui peut être substitué comme décrit ci-dessus pour un groupe aryle,

un groupe amino-alkyl($C_1$-$C_4$),

un groupe alcanoyl($C_1$-$C_4$)-amino-alkyle($C_1$-$C_4$),

un groupe aroyl($C_7$-$C_{13}$)-amino-alkyle($C_1$-$C_4$),

un groupe alcoxy($C_1$-$C_4$)-carbonylamino-alkyle($C_1$-$C_4$),

un groupe aryl($C_6$-$C_{12}$)-alcoxy($C_1$-$C_4$)-carbonylamino-alkyle-($C_1$-$C_4$),

un groupe aryl($C_6$-$C_{12}$)-alkyl($C_1$-$C_4$)-amino-alkyle ($C_1$-$C_4$),

un groupe alkyl($C_1$-$C_4$)-amino-alkyle($C_1$-$C_4$),

un groupe di-alkyl($C_1$-$C_4$)-amino-alkyl($C_1$-$C_4$),

un groupe guanidino-alkyle($C_1$-$C_4$),
un groupe imidazolyle, indolyle, un groupe alkyl($C_1$-$C_4$)-thio,
un groupe alkyl($C_1$-$C_4$)-thio-alkyle($C_1$-$C_4$), un groupe aryl($C_6$-$C_{12}$)-thio-alkyle($C_1$-$C_4$), qui peut être substitué dans la partie aryle, come décrit ci-dessus pour un groupe aryle, un groupe aryl($C_6$ $C_{12}$) alkyl($C_1$-$C_4$)-thio, qui peut être substitué dans la partie aryle, comme décrit ci-dessus pour un groupe aryle,
un groupe carboxy-alkyle($C_1$-$C_4$),
un groupe carboxy, carbamoyle,
un groupe carbamoyl-alkyle($C_1$-$C_4$),
un groupe alcoxy($C_1$-$C_4$)-carbonyl-alkyle($C_1$-$C_4$),
un groupe alcoxy($C_1$-$C_4$)-carbonyle,
un groupe aryl-oxy($C_1$-$C_{12}$)-alkyle($C_1$-$C_4$), qui peut être substitué dans la partie aryle, comme décrit ci-dessus pour un groupe aryle,
un groupe aryl($C_6$-$C_{12}$)-alcoxy($C_1$-$C_4$), qui peut être substitué dans la partie aryle, comme décrit ci-dessus pour un groupe aryle,
$R^1$ représente l'hydrogène,
un groupe alkyle ayant de 1 à 6 atomes de carbone,
un groupe alcényle ayant de 2 à 6 atomes de carbone,
un groupe alcynyle ayant de 2 à 6 atomes de carbone,
un groupe cycloalkoyle ayant de 3 à 9 atomes de carbone,
un groupe cycloalcényle ayant de 5 à 9 atomes de carbone,
un groupe cycloalkyl($C_3$-$C_9$)alkyle($C_1$-$C_4$),
un groupe cycloalkényle($C_5$-$C_9$)-alkyle($C_1$-$C_4$),
un groupe aryle éventuellement partiellement hydrogéné, ayant de 6 à 12 atomes de carbone, qui peut être substitué comme décrit ci-dessus pour R,
un groupe aryl($C_6$-$C_{12}$)-alkyle($C_1$-$C_4$) ou un groupe aroyl-($C_7$-$C_{13}$)-alkyle($C_1$ ou $C_2$),
qui peuvent être tous les deux substitués comme le groupe aryle précédent,
un groupe hétéroaryle mono- ou bicyclique, éventuellement partiellement hydrogéné, ayant de 5 à 7 ou de 8 à 10 atomes dans le noyau, dont de 1 à 2 atomes du noyau représentent des atomes de soufre ou d'oxygdne et/ou de 1 à 4 atomes du noyau représentent des atomes d'azote, qui peut être substitué comme le groupe aryle précédent, ou
la chaîne latérale d'un acide α-aminé d'origine naturelle R -CH($NH_2$)-COOH, éventuellement protégé,
$R^2$ et $R^3$ sont identiques ou différents et représentent l'hydrogène,
un groupe alkyle ayant de 1 à 6 atomes de carbone,
un groupe alcényle ayant de 2 à 6 atomes de carbone,
un groupe dialkyl($C_1$-$C_4$)-amino-alkyle($C_1$-$C_4$),
un groupe alcanoyloxy($C_1$-$C_5$)-alkyle($C_1$-$C_4$),
un groupe alcoxy($C_1$-$C_6$)-carbonyloxy-alkyle($C_1$-$C_4$),
un groupe aroyloxy($C_7$-$C_{13}$)-alkyle($C_1$-$C_4$),
un groupe aryloxy($C_6$-$C_{12}$)-carboryloxy-alkyle($C_1$-$C_4$),
un groupe aryle ayant de 6 à 12 atomes de carbone,
un groupe aryl($C_6$-$C_{12}$)-alkyle($C_1$-$C_4$),
un groupe cycloalkyle($C_3$-$C_9$), ou
un groupe cycloalkyl($C_3$-$C_9$)-alkyle($C_1$-$C_4$).

3. Procédé selon la revendication 1, caractérisé en ce que
n est 1 ou 2,
R représente un groupe alkyle($C_2$-$C_6$), alcényle($C_2$-$C_6$), cycloalkyle($C_3$-$C_9$), aminoalkyle($C_1$-$C_4$), acyl($C_2$-$C_5$)-amino-alkyle($C_1$-$C_4$), alcoxy($C_1$-$C_4$)-carbonylamino-alkyle($C_1$-$C_4$), aryl($C_6$-$C_{12}$)-alcoxy($C_1$-$C_4$)-carbonyl-amino-alkyle($C_1$-$C_4$),aroyl($C_7$-$C_{13}$)-amino-alkyle($C_1$-$C_4$), aryle($C_6$-$C_{12}$) qui peut être mono-, di- ou trisubstitué par un groupe alkyle($C_1$-$C_4$), alcoxy ($C_1$-$C_4$), hydroxy, halogéno, nitro, amino, alkyle ($C_1$-$C_4$)-amino, di-alkyl($C_1$-$C_4$)-amino, et/ou méthylénedioxy; ou
un groupe 3-indolyle,
$R^1$ représente l'hydrogène ou un groupe alkyle($C_1$-$C_6$), qui peut être substitué par un groupe amino, acyl ($C_1$-$C_6$)-amino ou benzoylamino; un groupe alcényle ($C_2$-$C_6$), cycloalcoyle($C_3$-$C_9$), cycloalcényle($C_5$-$C_9$), cycloalkyl($C_3$-$C_7$)-alkyle($C_1$-$C_4$), aryle($C_6$-$C_{12}$) ou aryle partiellement hydrogéné qui peuvent être chacun substitués par un groupe alkyle($C_1$-$C_4$), alcoxy en ($C_1$ ou $C_2$) ou un halogène; un groupe aryl ($C_6$-$C_{12}$)-alkyle-($C_1$-$C_4$), ou aroyl($C_7$-$C_{13}$)-alkyle ($C_1$-$C_2$) qui peuvent être tous les deux substitués dans la partie aryle comme précédemment défini; un groupe hétérocyclique mono- ou bicyclique ayant de 5 à 7 ou de 8 à 10 atomes dans le noyau, dont de 1 à 2 atomes du noyau représentent des atomes d'oxygène ou de soufre et/ou de 1 à 4 atomes du noyau représentent des atomes d'azote; ou une chaîne latérale d'un acide α-aminé, d'origine naturelle, éventuellement protégé.
$R_2$ et $R_3$ sont identiques ou différents et représentant l'hydrogène, un groupe alkyle($C_1$-$C_6$), alcényle($C_2$-$C_6$) ou un groupe aryl($C_6$-$C_{12}$)-alkyle($C_1$-$C_4$).

4. Procédé selon la revendication 1, caractérisé en ce que
n est 1 ou 2,
R représente un groupe éthyle, alcoxy($C_1$-$C_4$)-carbo-nylamino-alkyle($C_1$-$C_4$), aryl($C_6$-$C_{12}$)-alcoxy($C_1$-$C_4$)-

carbonylamino-alkyle($C_1$-$C_4$), cyclohexyle ou phényle, qui peut être mono- ou disubstitué, ou dans le cas d'un groupe méthoxy, trisubstitué par un groupe phényle, alkyle($C_1$ ou $C_2$), alcoxy($C_1$ ou $C_2$), hydroxy, fluoro, chloro, bromo, amino, alkyl-($C_1$-$C_4$)-amino, di-alkyl($C_1$-$C_4$)-amino, nitro et/ou méthylénedioxy,

$R^1$ représente l'hydrogène, un groupe alkyle($C_1$-$C_3$), alcényle($C_2$ ou $C_3$), la chaîne latérale éventuellement protégée de la lysine, un groupe benzyle, 4-méthoxybenzyle, 4-éthoxybenzyle, phénéthyle, 4-amino-butyle ou benzoylméthyle et

$R^2$ et $R^3$ sont identiques ou differents et representent 1-hydrogdne, un groupe alkyle($C_1$-$C_4$) ou benzyle.

5. Composé de formule II

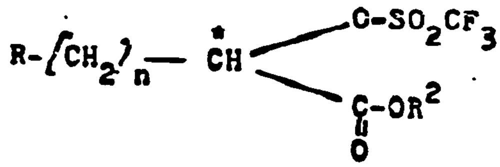

(II)

dans laquelle

n est 2,

R représente un groupe alkyle($C_1$-$C_6$), alcényl($C_2$ $C_6$), cyclo-alkyle($C_3$-$C_9$), acyl($C_2$-$C_5$)-amino-alkyle($C_1$-$C_4$), aroyl-($C_7$-$C_{13}$)-amino-alkyle($C_1$-$C_4$), alcoxy ($C_1$-$C_4$)-carbonylamino-alkyle($C_1$-$C_4$), aryl($C_6$-$C_{12}$)-alcoxy($C_1$-$C_4$)-carbonylamino-alkyle($C_1$-$C_4$), aryle ($C_6$-$C_{12}$) qui peut étre mono-, di- ou trisubstitué par un groupe alkyle($C_1$-$C_4$), alcoxy($C_1$-$C_4$), hydroxy, halogéno, nitro, amino, alkyl($C_1$-$C_4$)-amino, di-alkyl($C_1$-$C_4$)-amino et/ou méthylènedioxy; ou un groupe 3-indolyle et

$R^2$ représente l'hydrogène, un groupe alkyle($C_1$-$C_6$), alcényle- en ($C_2$-$C_6$) ou aryl($C_6$-$C_{12}$)-alkyle($C_1$-$C_4$).

6. Composé de formule III

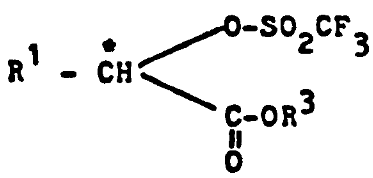

(III)

dans laquelle

$R^1$ représente l'hydrogène, ou un groupe alkyle($C_2$-$C_6$) qui peut être substitué éventuellement par un groupe amino, acyl-($C_1$-$C_6$)-amino ou benzoylamino; un groupe alcényle en ($C_2$-$C_6$), cycloalkyle($C_3$-$C_9$), cycloalcényle en ($C_5$-$C_9$), cycloalkyle-($C_3$-$C_7$-al-kyle($C_1$-$C_4$), aryle($C_6$-$C_{12}$) ou aryle partiellement hydrogéné, qui peuvent être chacun subsitué par un groupe alkyle en ($C_1$-$C_4$), alcoxy($C_1$ ou $C_2$) ou un halogène; un groupe aryl($C_6$-$C_{12}$)-alkyle($C_2$-$C_4$) ou aroyl($C_7$-$C_{13}$)-alkyle($C_1$-$C_2$) qui peuvent être tous les deux substitués, comme défini précédemment, dans le groupe aryle; un groupe hétérocyclique mono- ou bicycliqye ayant de 5 à 7 ou de 8 à 10 atomes dans le noyau, dont de 1 à 2 atomes du noyau représentent des atomes de soufre ou d'oxygène et/ou de 1 à 4 atomes du noyau sont des atomes d'azote, ou une chaîne latérale d'un acide α-aminé d'origine naturelle, protégée, et

$R^3$ représente l'hydrogène, un groupe alkyle($C_1$ $C_6$), alcényle($C_2$-$C_6$) ou aryl($C_6$-$C_{12}$)-alkyle($C_1$-$C_4$).

7. Compose selon la revendication 5, caractérisé en ce que

R représente un groupe éthyle, tert-butoxycarbonyl-amino-alkyle($C_1$-$C_4$), benzyloxycarbonylamino-alky-le($C_1$-$C_4$), cyclohexyle ou phényle qui peut être mono- ou di-substitué ou, dans le cas d'un groupe méthoxy, trisubstitué par un groupe phényle, alkyle ($C_1$ ou $C_2$), alcoxy($C_1$ ou $C_2$), hydroxy, fluoro, chloro, bromo, amino, alkyl($C_1$-$C_4$)-amino, di- al-kyl($C_1$-$C_4$)-amino, nitro et/ou méthylénedioxy et

$R^2$ représente l'hydrogène, un groupe alkyle($C_1$-$C_4$) ou benzyle.

8. Composé selon la revendication 6, caractérisé en ce que

$R^1$ représente l'hydrogène, un groupe alkyle($C_2$ ou $C_3$), alcényle($C_2$ ou $C_3$), la chaîne latérale protégée de la lysine, un groupe 4-méthoxybenzyle, 4-éthoxy-benzyle, phénétyle, 4-aminobutyle ou benzoylméthyle, et

$R^3$ représente l'hydrogène, un groupe alkyle ($C_1$-$C_4$) ou benzyle.

9. Composé selon la revendication 5, caractérisé en ce que
R représente un groupe phényle ou un groupe phényle mono- ou disubstitué avec le fluor et/ou le chlore, et
$R^2$ représente l'hydrogène, un groupe méthyle, éthyle, tert-butyle ou benzyle.

10. Composé selon la revendication 6, caractérisé en ce que
$R^1$ représente la chaîne latérale acylée de la lysine ou la chaîne latérale O-alcoylée($C_1$-$C_6$) de la tyrosine et
$R^3$ représente l'hydrogène, un groupe méthyle, éthyle, tert-butyle ou benzyle.

11. Procédé pour la préparation de composés de formules II ou III, caractérisé en ce qu'on fait réagir des dérivés d'acides α-hydroxycarboxyliques de formule VI ou VII

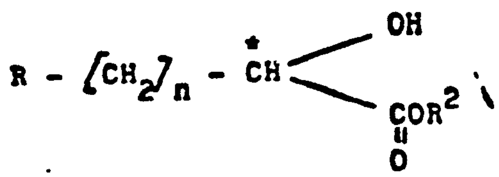

(VI)

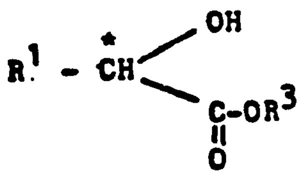

(VII)

dans lesquelles n, R, R, $R^2$ et $R^3$ ont les significations indiquées ci-dessus, avec un agent de trifluorométhane sulfonation, éventuellement en présence d'une base.

**Revendications** pour l'Etat contractant: AT

1. Procédé pour la préparation de composés de formule I

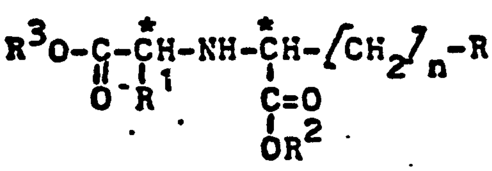

(I)

dans laquelle
n est 1 ou 2,
R représente l'hydrogène,
un groupe carboxy, carbamoyle,
un groupe aliphatique éventuellement substitué, ayant de 2 à 8 atomes de carbone,
un groupe cycloaliphatique éventuellement substitué, ayant de 3 à 9 atomes de carbone,
un groupe aromatique éventuellement substitué ayant de 6 à 12 atomes de carbone,
un groupe $OR^4$ ou $OS^4$, dans lequel
$R^4$ représente un groupe aliphatique éventuellement substitué ayant de 1 à 4 atomes de carbone, un groupe aromatique éventuellement substitué ayant de 6 à 12 atomes de carbone, ou un groupe hétéroaromatique éventuellement substitué ayant de 5 à 10 atomes dans le noyau;
ou
un groupe hétéroaromatique éventuellement substitué ayant de 5 à 10 atomes dans le noyau,
$R^1$ représente l'hydrogène,
un groupe aliphatique éventuellement substitué ayant de 1 à 6 atomes de carbone,
un groupe cycloaliphatique éventuellement substitué ayant de 3 à 9 atomes de carbone,
un groupe cycloaliphatique-aliphatique éventuellement substitué, ayant de 4 à 13 atomes de carbone,
un groupe aromatique éventuellement substitué ayant de 6 à 12 atomes de carbone,
un groupe araliphatique éventuellement substitué ayant de 7 à 16 atomes de carbone,

un groupe hétéroaromatique éventuellement substitué ayant de 5 à 10 atomes de carbone ou la chaîne latérale d'un acide α-aminé d'origine naturelle, éventuellement protégé,

$R^2$ et $R^3$ sont identiques ou différents et représentent l'hydrogène,
un groupe aliphatique éventuellement substitué ayant de 1 à 6 atomes de carbone,
un groupe cycloaliphatique éventuellement substitué ayant de 3 à 9 atomes de carbone,
un groupe cycloaliphatique-aliphatique éventuellement substitué ayant de 4 à 12 atomes de carbone,
un groupe aromatique éventuellement substitué ayant de 6 à 12 atomes de carbone ou
un groupe araliphatique éventuellement substitué, ayant de 7 à 16 atomes de carbone,
ce procédé étant caractérisé en ce qu'on fait réagir des composés de formules II ou III

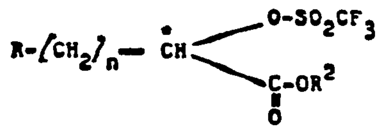
(II)

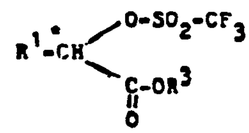
(III)

dans lesquelles n, R, $R^1$, $R^2$ et $R^3$ ont les significations indiquées ci-dessus, avec des composés de formules IV ou V,

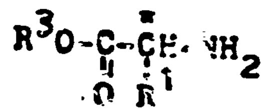
(IV)

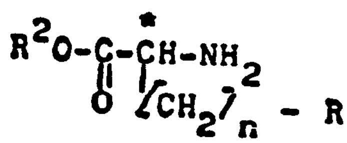
(V).

dans lesquelles R, $R^1$, $R^2$, $R^3$ et n ont les significations indiquées ci-dessus, on élimine éventuellement les groupes ester par hydrolyse ou hydrogénolyse ou bien on estérifie éventuellement les groupes carboxy libres d'une manière connue en soi.

2. Procédé selon la revendication 1, caractérisé en ce que
n est 1 ou 2,
R représente l'hydrogène,
un groupe alkyle ayant de 2 à 8 atomes de carbone, un groupe alcényle ayant de 2 à 6 atomes de carbone,
un groupe cycloalkyle ayant de 3 à 9 atomes de carbone,
un groupe aryle ayant de 6 à 12 atomes de carbone, qui peut être mono-, di- ou trisubstitué par un groupe alkyle($C_1$-$C_4$), alcoxy($C_1$-$C_4$), hydroxy, halogéno, nitro, amino, aminométhyl, alkyl($C_1$-$C_4$)-amino, di-alkyl-($C_1$-$C_4$)-amino, acyl($C_1$-$C_4$)-amino, de préférence alcanoyl-($C_1$-$C_4$)-amino, méthylènedioxy, carboxy, cyano et/ou sulfamoyle,
un groupe alcoxy ayant de 1 à 4 atomes de carbone,
un groupe aryloxy ayant de 6 à 12 atomes de carbone,
qui peut être substitué comme décrit ci-dessus pour un groupe aryle,
un groupe hétéroaryloxy mono- ou bicyclique ayant de 5 à 7 ou de 8 à 10 atomes dans le noyau, dont de 1 à 2 atomes du noyau représentent des atomes d'oxygène ou de soufre et/ou de 1 à 4 atomes du noyau représentent des atomes d'azote,
qui peut être substitué comme décrit ci-dessus pour un groupe aryle,
un groupe amino-alkyle(C -$C_4$),
un groupe alcanoyl($C_1$-$C_4$)-amino-alkyle($C_1$-$C_4$),
un groupe aroyl($C_7$-$C_{13}$)-amino-alkyle($C_1$-$C_4$),
un groupe alcoxy($C_1$-$C_4$)-carbonylamino-alkyle($C_1$-$C_4$),
un groupe aryl($C_6$-$C_{12}$)-alcoxy($C_i$-$C_4$)-carbonyl-amino-alkyle($C_1$-$C_4$),
un groupe aryl($C_6$-$C_{12}$)-alkyl(C -$C_4$)-amino-alkyl ($C_1$-$C_4$),
un groupe alkyl($C_1$-$C_4$)-amino-alkyle($C_1$-$C_4$),
un groupe di-alkyl($C_1$-$C_4$)-amino-alkyle($C_1$-$C_4$),
un groupe guanidino-alkyle($C_1$-$C_4$),
un groupe imidazolyle, indolyle,
un groupe alkyl($C_1$-$C_4$)-thio,

un groupe alkyl($C_1$-$C_4$)thio-alkyle($C_1$-$C_4$),
un groupe aryl($C_6$-$C_{12}$)-thio-alkyle($C_1$-$C_4$),
qui peut être substitué dans la partie aryle comme décrit ci-dessus pour un groupe aryle,
un groupe aryl($C_6$-$C_{12}$)-alkyl($C_1$-$C_4$)-thio,
qui peut être substitué dans la partie aryle, comme décrit ci-dessus pour un groupe aryle,
un groupe carboxy-alkyle($C_1$-$C_4$),
un groupe carboxy, carbamoyle,
un groupe carbamol-alkyle($C_1$-$C_4$),
un groupe alcoxy($C_1$-$C_4$)-carbonyl-alkyle($C_1$-$C_4$),
un groupe alcoxy($C_1$-$C_4$)-carbonyle,
un groupe aryloxy($C_1$-$C_{12}$)-alkyle($C_1$-$C_4$),
qui peut être substitué dans la partie aryle, comme décrit ci-dessus pour un groupe aryle,
un groupe aryl($C_6$-$C_{12}$)-alcoxy($C_1$-$C_4$), qui peut être substitué dans la partie aryle, comme décrit ci-dessus pour un groupe aryle,
$R^1$ représente l'hydrogène,
un groupe alkyle ayant de 1 à 6 atomes de carbone,
un groupe alcényle ayant de 2 à 6 atomes de carbone,
un groupe alcynyle ayant de 2 à 6 atomes de carbone,
un groupe cycloalkyle ayant de 3 à 9 atomes de carbone,
un groupe cycloalcényle ayant de 5 à 9 atomes de carbone,
un groupe cycloalkyl($C_3$-$C_9$)-alkyle($C_1$-$C_4$),
un groupe cycloalcényl($C_5$-$C_9$)-alkyle($C_1$-$C_4$),
un groupe aryle éventuellement partiellement hydrogéné, ayant de 6 à 12 atomes de carbone,
qui peut être substitué tel que décrit pour R,
un groupe aryl($C_6$-$C_{12}$)-alkyle($C_1$-$C_4$) ou un groupe aroyl-($C_7$-$C_{13}$)alkyle($C_1$ ou $C_2$),
qui peuvent tous les deux être substitués, comme pour le groupe aryle précédent,
un groupe hétéroaryle mono- ou bicyclique, éventuellement partiellement hydrogéné, ayant de 5 à 7 ou de 8 à 10 atomes dans le noyau, dont de 1 à 2 atomes du noyau représentent des atomes d'oxygène ou de soufre et/ou de 1 à 4 atomes du noyau représentent des atomes d'azote,
qui peut être substitué comme le groupe aryle précédent, ou
la chaîne latérale d'un acide α-aminé d'origine naturelle $R^1$-$CH(NH_2)$-COOH, éventuellement protégé,
$R^2$ et $R^3$ sont identiques ou différents et représentent l'hydrogène,
un groupe alkyle ayant de 1 à 6 atomes de carbone,
un groupe alcényle ayant de 2 à 6 atomes de carbone,
un groupe di-alkyl($C_1$-$C_4$)-amino-alkyle($C_1$-$C_4$),
un groupe alcanoyloxy($C_1$-$C_5$)-alkyle($C_1$-$C_4$),
un groupe alcoxy($C_1$-$C_6$)-carbonyloxy-alkyle($C_1$-$C_4$),
un groupe aroyloxy($C_7$-$C_{13}$)-alkyle($C_1$-$C_4$),
un groupe aryloxy($C_6$-$C_{12}$)-carbonyloxy-alkyle($C_1$-$C_4$),
un groupe aryle ayant de 6 à 12 atomes de carbone,
un groupe aryl($C_6$-$C_{12}$)-alkyle($C_1$-$C_4$),
un groupe cycloalkyle($C_3$-$C_9$) ou
un groupe cycloalkyl($C_3$-$C_9$)-alkyle($C_1$-$C_4$).

3. Procédé selon la revendication 1, caractérisé en ce que
n est 1 ou 2,
R représente un groupe alkyle($C_2$-$C_6$), alcényle ($C_2$-$C_6$), cycloalkyle($C_3$-$C_9$), amino-alkyle($C_1$-$C_4$), acyl($C_2$-$C_5$)-amino-alkyle($C_1$-$C_4$), alcoxy($C_1$-$C_4$)-carbonylamino-alkyle($C_1$-$C_4$), aryl($C_6$-$C_{12}$)-alcoxy ($C_1$-$C_4$)-carbonylamino-alkyle($C_1$-$C_4$), aroyl($C_7$-$C_{13}$)-amino-alkyle($C_1$-$C_4$), aryle($C_6$-$C_{12}$), qui peut être mono-, di- ou trisubstitué par un groupe al-kyle($C_1$-$C_4$), alcoxy($C_1$-$C_4$), hydroxy, halogéno, nitro, amino, alkyl($C_1$-$C_4$)-amino, di-alkyl($C_1$-$C_4$)-amino et/ou méthylènedioxy;
ou un groupe 3-indolyle,
$R^1$ représente l'hydrogène ou un groupe alkyle($C_1$-$C_6$) qui peut être éventuellement substitué par un groupe amino, acyl($C_1$-$C_6$)-amino ou benzoylamino; un groupe alcényle($C_2$-$C_6$), cycloalkyle($C_3$-$C_9$), cycloalcényle($C_5$-$C_9$), cycloalkyl-($C_3$-$C_7$)-alkyle ($C_1$-$C_4$), aryle($C_6$-$C_{12}$) ou aryle partiellement hydrogéné, qui peuvent être chacun substitués par un groupe alkyle($C_1$-$C_4$), alcoxy($C_1$ ou $C_2$) ou par un halogène; un groupe aryl($C_6$-$C_{12}$)-alkyle($C_1$-$C_4$) ou aroyl($C_7$-$C_{13}$)-alkyle-($C_1$-$C_2$) qui peuvent être tous les deux substitués comme défini cidessus pour un groupe aryle; un groupe hétérocyclique mono- ou bicyclique ayant de 5 à 7 ou de 8 à 10 atomes dans le noyau, dont de 1 à 2 atomes du noyau sont des atomes de soufre ou d'oxygdne et/ou de 1 à 4 atomes du noyau sont des atomes d'azote; ou une chaîne latérale d'un acide α-aminé d'origine naturelle, éventuellement protégé,
$R^2$ et $R^3$ sont identiques ou différents et représentent l'hydrogène, un groupe alkyle($C_1$-$C_6$), alcényle ($C_2$-$C_6$) ou aryl($C_6$-$C_{12}$)-alkyle($C_1$-$C_4$).

4. procédé selon la revendication 1, caractérisé en ce que
n est 1 ou 2,
R représente un groupe éthyle, alcoxy($C_1$-$C_4$)-carbonylamino-alkyle($C_1$-$C_4$), aryl($C_6$-$C_{12}$)-alcoxy($C_1$-$C_4$)-carborylamino-alkyle($C_1$-$C_4$), cyclohexyle ou phényle, qui peut être mono-ou disubstitué ou, dans le cas d'un

groupe méthoxy, trisubstitué par un groupe phényle, alkyle($C_1$ ou $C_2$), alcoxy($C_1$ ou $C_2$), hydroxy, fluoro, chloro, bromo, amino, alkyl ($C_1$-$C_4$)-amino, di-alkyl($C_1$-$C_4$)-amino, nitro et/ou méthylènedioxy,

$R^1$ représente l'hydrogène, un groupe alkyle($C_1$-$C_3$), alcényle-($C_2$ ou $C_3$), la chaîne latérale éventuellement protégée de la lysine, un groupe benzyle, 4-méthoxybenzyle, 4-éthoxybenzyle, phénéthyle, 4-amino-butyle ou benzoylmgthyle, et

$R^2$ et $R^3$ sont identiques ou différents et représentent l'hydrogène, un groupe alkyle($C_1$-$C_4$) ou benzyle.

5. Procédé pour la préparation de composés de formule II

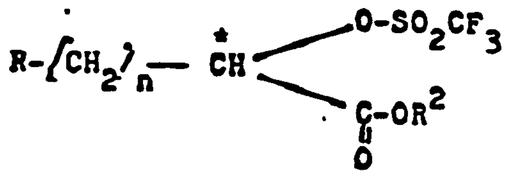

(II)

dans laquelle

n est 2,

R représente un groupe alkyle($C_1$-$C_6$), alcényle($C_2$-$C_6$), cycloalkyle($C_3$-$C_9$), acyl($C_2$-$C_5$)-amino-alkyle ($C_1$-$C_4$), aroyl($C_7$-$C_{13}$)-amino-alkyle($C_1$-$C_4$), alcoxy($C_1$-$C_4$)-carbonyl-amino-alkyle($C_1$-$C_4$), aryl ($C_6$-$C_{12}$)-alcoxy($C_1$-$C_4$)-carbonyl-amino-alkyle($C_1$-$C_4$), aryle($C_6$-$C_{12}$), qui peut être mono- di- ou trisubstitué par un groupe alkyle($C_1$-$C_4$), alcoxy-($C_1$-$C_4$), hydroxy, halogéno, nitro, amino, alkyl ($C_1$-$C_4$)-amino, di-alkyl($C_1$-$C_4$)-amino et/ou méthylènedioxy; ou un groupe 3-indolyle et

$R^2$ représente l'hydrogène, un groupe alkyle($C_1$-$C_6$), alcényle-($C_2$-$C_6$) ou aryl($C_6$-$C_{12}$)-alkyle($C_1$-$C_4$), caractérisé en ce qu'on fait réagir un dérivé d'acide α-hydroxy-carboxylique de formule VI,

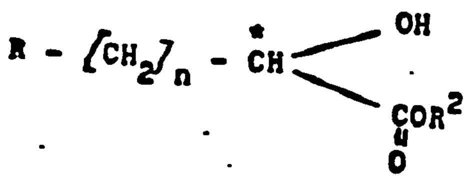

(VI)

dans laquelle n, R et $R^2$ ont les significations indiquées ci-dessus, avec un agent de trifluorométhane sulfonation éventuellement en présence d'une base.

6. Procédé pour la préparation de composés de formule III

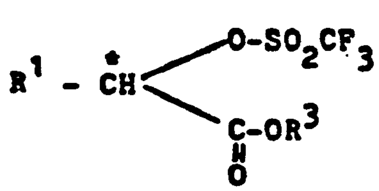

(III)

dans laquelle

$R^1$ représente l'hydrogène ou un groupe alkyle($C_2$-$C_6$) qui peut éventuellement être substitué par un groupe amino, acyl($C_1$-$C_6$)-amino ou benzoylamino; un groupe alcényle($C_2$-$C_6$), cycloalkyle($C_3$-$C_9$), cycloalcényle($C_5$-$C_9$), cycloalkyl($C_3$-$C_7$)-alkyle ($C_1$-$C_4$), aryle($C_6$-$C_{12}$) ou aryle partiellement hydrogéné qui peuvent être substitués chacun par un groupe alkyle($C_1$-$C_4$), alcoxy($C_1$ ou $C_2$) ou par un halogène; un groupe aryl($C_6$-$C_{12}$)-alkyle($C_2$-$C_4$) ou aroyl($C_7$-$C_{13}$)-alkyle($C_1$-$C_2$) qui peuvent tous les deux être substitués dans le groupe aryle, comme défini précédemment; un groupe hétérocyclique mono- ou bicyclique ayant de 5 à 7 ou de 8 à 10 atomes dans le noyau, dont de 1 à 2 atomes du noyau représentent des atomes de soufre ou d'oxygène et/ou de 1 à 4 atomes

du noyau représentent des atomes d'azote; ou une chaîne latérale d'un acide α-aminé d'origine naturelle, protégé et

$R^3$ représente l'hydrogène, un groupe alkyle($C_1$ $C_6$), alcényle-($C_2$-$C_6$)ou aryl($C_6$-$C_{12}$)-alkyle($C_1$-$C_4$), caractérisé en ce qu'on fait réagir un dérivé d'acide α-hydroxy-carboxylique de formule VII

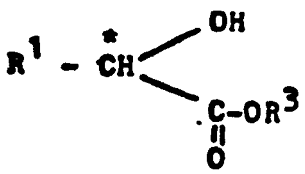

(VII)

dans laquelle $R^1$ et $R^3$ ont les significations indiquées ci-dessus, avec un agent de trifluorométhane sulfonation, éventuellement en présence d'une base.

7. Procédé selon la revendication 5, caractérisé en ce que

R représente un groupe éthyle, tert-butoxycarbonyl-amino-alkyle($C_1$-$C_4$), benzyloxycarbcnylamino-alkyle ($C_1$-$C_4$), cyclohexyle ou phényle qui peut être mono- ou disubstitué, ou, dans le cas d'un groupe méthoxy,trisubstitué par un groupe phényle, al-kyle($C_1$-$C_2$), alcoxy($C_1$-$C_2$), hydroxy, fluoro, chloro, bromo, amino, alkyl($C_1$-$C_4$)-amino, di-alkyl ($C_1$-$C_4$)-amino, nitro et/ou méthylènedioxy, et

$R^2$ représente l'hydrogène, un groupe alkyle($C_1$-$C_4$) ou benzyle.

8. Procédé selon la revendication 6, caractérisé en ce que

$R^1$ représente l'hydrogàne, un groupe alkyle($C_2$-$C_3$), alcényle-($C_2$-$C_3$), la chaîne latérale protégée de la lysine, un groupe 4-méthoxybenzyle, 4-éthoxy-benzyle, phénéthyle, 4-amino-butyle ou benzoylméthyle et

$R^3$ représente l'hydrogène, un groupe alkyle($C_1$-$C_4$) ou benzyle.

9. Procédé selon la revendication 5, caractérisé en ce que

R représente un groupe phényle ou un groupe phényle mono- ou disubstitué par le fluor et/ou le chlore et

$R^2$ represente l'hydrogène, un groupe méthyle, éthyle, tert-butyle ou benzyle.

10. Procédé selon la revendication 6, caractérisé en ce que

$R^1$ représente la chaîne latérale acylée de la lysine ou la chaîne latérale O-alkylée($C_1$-$C_6$) de la tyrosine et

$R_3$ représente l'hydrogène, un groupe méthyle, éthyle, tert-butyle ou benzyle.